(19) Europäisches Patentamt / European Patent Office / Office européen des brevets

(11) EP 2 787 960 B1

# (12) FASCICULE DE BREVET EUROPEEN

(45) Date de publication et mention de la délivrance du brevet:
**06.09.2017 Bulletin 2017/36**

(21) Numéro de dépôt: **12810343.9**

(22) Date de dépôt: **05.12.2012**

(51) Int Cl.:
***A61K 8/73*** *(2006.01)* ***A61K 8/81*** *(2006.01)*
***A61K 8/06*** *(2006.01)* ***A61Q 5/00*** *(2006.01)*
***A61Q 19/00*** *(2006.01)*

(86) Numéro de dépôt international:
**PCT/FR2012/052802**

(87) Numéro de publication internationale:
**WO 2013/083912 (13.06.2013 Gazette 2013/24)**

## (54) NOUVELLES ÉMULSIONS HUILE-DANS-EAU RICHES EN SELS, À VISCOSITÉ ÉLEVÉE ET STABLES AU COURS DU TEMPS

NEUE MIT SALZ ANGEREICHERTE, HOCHVISKOSE UND LANGZEITSTABILE ÖL-IN-WASSER-EMULSIONEN

NOVEL OIL-IN-WATER EMULSIONS ENRICHED WITH SALT WHICH ARE HIGHLY VISCOUS AND STABLE OVER TIME

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.12.2011 FR 1161303**

(43) Date de publication de la demande:
**15.10.2014 Bulletin 2014/42**

(73) Titulaire: **Societe D'Exploitation De Produits Pour Les Industries Chimiques Seppic**
**75007 Paris (FR)**

(72) Inventeur: **MERAT, Emmanuelle**
**F-81440 Lautrec (FR)**

(74) Mandataire: **Conan, Philippe Claude**
**L'Air Liquide SA**
**Direction de la Propriété Intellectuelle**
**75, quai d'Orsay**
**75321 Paris Cedex 07 (FR)**

(56) Documents cités:
**EP-A1- 0 152 095**
**WO-A1-2011/030044**
**FR-A1- 2 466 273**
**FR-A1- 2 940 111**
**US-A1- 2011 274 629**

- **P. BERNARD ET AL: "A new polymer with a maximum resistance to electrolytes", SÖFW-JOURNAL, vol. 136, no. 12, 1 décembre 2010 (2010-12-01), pages 55-58, XP055024800,**
- **BERNARD P: "Seppic's polymer with a maximum resistance to electrolytes", INTERNET CITATION, 1 mars 2010 (2010-03-01), pages 1-22, XP002674160, Extrait de l'Internet: URL:http://lica.com.tw/english/licadata/seppic/SepiMAX%20ZEN [extrait le 2012-04-18]**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet de nouvelles émulsions huile-dans-eau, ainsi que leurs utilisations en cosmétique et en pharmacie.

Les compositions cosmétiques se présentant sous la forme d'émulsions huile-dans-eau commercialisées par l'industrie cosmétique et par l'industrie pharmaceutique comprennent très régulièrement des polymères épaississants synthétiques pour augmenter la viscosité desdites émulsions huile-dans-eau qui peuvent se présenter sous la forme de crèmes, de laits, et qui sont appliquées directement sur la peau.

Ces polymères épaississants synthétiques permettent d'épaissir les phases aqueuses présentes dans lesdites émulsions huile-dans-eau, procurant ainsi soit l'obtention de la consistance désirée soit un effet de stabilisation de ladite émulsion.

Les polymères épaississants synthétiques actuellement utilisés en ces domaines, se présentent sous deux formes physiques, la forme poudre et la forme liquide pour laquelle le polymère est préparé par polymérisation radicalaire en émulsion inverse à l'aide d'agents tensioactifs, et que l'on appelle couramment latex inverse.

Parmi les polymères épaississants synthétiques se présentant sous la forme d'une poudre les plus connus, on peut citer les polymères à base d'acide acrylique ou les copolymères à base d'acide acrylique et de ses esters. On citera par exemple, les polymères commercialisés sous le nom de marque, CARBOPOL™ et PEMULEN™. Ils sont décrits notamment dans les brevets américains US 5,373,044, US 2,798,053 et dans le brevet européen EP 0 301 532.

En cosmétique, on utilise aussi et toujours sous forme de poudre, des homopolymères ou des copolymères à base d'acide 2-acrylamido-2-méthyl-propane sulfonique et/ou de ses sels. Ces polymères épaississants sont commercialisés sous le nom de marque Aristoflex™ et décrits notamment dans les brevets européens EP 816 403, EP 1 116 733 et EP 1 069 142. Ces épaississants synthétiques sous forme de poudre sont obtenus par polymérisation précipitante ; le (ou les) monomère(s) est (ou sont) mis en solution dans un solvant organique type benzène, acétate d'éthyle, cyclohexane, tertio-butanol ; ce procédé nécessite donc de nombreuses étapes successives de purification du produit final, pour éliminer toute trace de solvant résiduel.

Les industries de la cosmétique et de la pharmacie utilisent aussi très largement des épaississants se présentant sous la forme de latex inverses et notamment ceux commercialisés par la demanderesse. On citera par exemple, les épaississants Sepigel™ 305, Simulgel™ 600, Simulgel™ EG, Simulgel™ EPG, Simulgel™ NS, Simulgel™ A, Sepiplus™ 400, Sepiplus™ 250 et Sepiplus™ 265. Ces épaississants sont obtenus par polymérisation radicalaire en émulsion inverse. Ils présentent l'avantage d'être plus aisément manipulables, notamment à température ambiante, et se dispersent très rapidement dans l'eau. De plus, ces produits développent des performances épaississantes remarquablement élevées ; ces performances sont probablement la conséquence du procédé mis en oeuvre pour leur préparation, une réaction de polymérisation radicalaire en phase dispersée, qui conduit à des polymères de très hauts poids moléculaires.

Néanmoins, ces épaississants synthétiques se présentant sous la forme de latex inverse contiennent une huile, et un ou plusieurs agents tensioactifs qui peuvent parfois induire des réactions d'intolérance cutanée sur des sujets particulièrement sensibles ; de plus cette présence d'huile les rend inutilisables pour la préparation de gels aqueux clairs.

La demanderesse a donc développé des épaississants synthétiques ayant les performances épaississantes équivalentes ou supérieures aux latex inverses, mais mieux tolérés par la peau, en particulier du fait de l'absence de toute phase huile pouvant conduire à des gels aqueux plus clairs. Ces produits se présentent sous la forme de poudre mais possèdent des temps de dissolution, et donc une facilité de mise en oeuvre, comparable à ceux des produits se présentant sous la forme de liquides. Ces composés, décrits dans la demande de brevet européen publiée sous le numéro EP 1 496 081, sont obtenus par les techniques classiques de polymérisation, telles que la polymérisation radicalaire en phase dispersée, la polymérisation radicalaire en suspension inverse, la polymérisation radicalaire en émulsion inverse ou en microémulsion inverse. Les systèmes épaississants synthétiques obtenus sont ensuite extraits et purifiés par différentes techniques telles que la précipitation dans un tiers solvant, la précipitation dans un tiers solvant suivie éventuellement d'un lavage, d'un séchage par atomisation ou par déshydratation azéotropique, suivie éventuellement d'un lavage par un solvant judicieusement choisi. Ces épaississants synthétiques combinent donc une partie des avantages des épaississants synthétiques se présentant sous la forme de poudres classiques (absence d'huile, obtention de gels aqueux plus clairs) et les avantages des épaississants synthétiques se présentant sous la forme de latex inverse (haute vitesse de dissolution, pouvoir épaississant et propriétés stabilisantes remarquables). Cependant pour certaines utilisations, les clients utilisateurs de tels systèmes épaississants synthétiques souhaitent pouvoir fabriquer des gels encore plus clairs, que ceux obtenus aujourd'hui, voir des gels transparents. De plus, les gels obtenus avec ces épaississants synthétiques n'ont pas une stabilité satisfaisante lorsque la composition est riche en électrolytes comme c'est souvent le cas des compositions comprenant des filtres solaires et/ou des pigments colorés et/ou des extraits de végétaux riches en électrolytes.

La demanderesse a aussi développé des systèmes épaississants synthétiques comme ceux décrits dans la demande brevet français publiée sous le numéro 2 910 899, qui divulgue un terpolymère linéaire, branché ou réticulé d'au moins un monomère possédant une fonction acide fort, libre, partiellement salifiée ou totalement salifiée, avec au

moins un monomère neutre, et au moins un monomère de formule (A) :

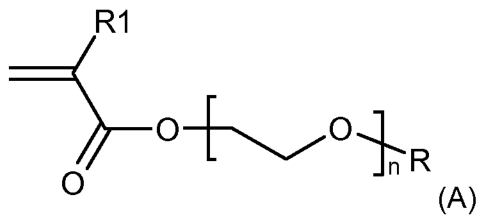

(A)

dans laquelle R1 représente un atome d'hydrogène ou un radical méthyle, R représente un radical alkyle linéaire ou ramifié comportant de huit à trente atomes de carbone et n représente un nombre supérieur ou égal à un et inférieur ou égal à cinquante. Ces polymères présentent des propriétés épaississantes très prononcées, notamment en présence d'électrolytes. Ils fonctionnent sur une large gamme de pH et permettent de réaliser des gels transparents. Cependant, les formulations à bas pH épaissies par certains d'entre eux n'ont pas une tenue aux sels satisfaisante sur le long terme et certaines d'entres elles, qui contiennent des alcools gras, ont un aspect élastique peu engageant et donnant des sensations de collant au toucher et/ou un aspect d'une crème ou d'une émulsion granuleuse et non continues.

La demanderesse a mis en évidence que ces inconvénients pouvaient être évités en sélectionnant certains de ces terpolymères, qui n'avaient pas été divulgués dans la demande de brevet français publiée sous le numéro 2 910 899, et a développé de nouveaux polyélectrolytes anioniques branchés ou réticulés, comme ceux décrits dans la demande internationale publiée sous le numéro WO 2011/030044, qui sont issus de la polymérisation radicalaire d'au moins un monomère possédant une fonction acide fort, partiellement salifiée ou totalement salifiée, avec au moins un monomère neutre, et au moins un monomère de formule (B) :

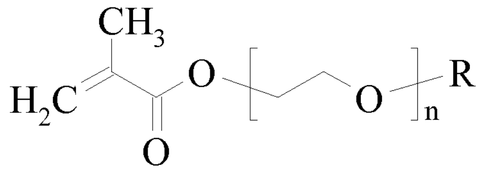

(B)

dans laquelle R représente un radical alkyle linéaire ou ramifié comportant de huit à vingt atomes de carbone et n représente un nombre supérieur ou égal à un et inférieur ou égal à trente.

Les industries de la cosmétique et de la pharmacie sont également à la recherche de formes galéniques réduisant les risques d'intolérances cutanées, et par conséquent tendent à sélectionner des ingrédients constitutifs desdites formes galéniques qui soient bien tolérés et également à diminuer, dans leur constitution, la part d'ingrédients susceptibles d'augmenter la probabilité de réactions d'intolérances sur la peau. A cet égard, les industries de la cosmétique et de la pharmacie cherchent à mettre au point des émulsions huile-dans-eau qui soient dépourvues de systèmes stabilisants comprenant des tensio-actifs émulsionnants. Les terpolymères décrits dans la demande internationale publiée sous le numéro WO 2011/030044 constituent donc des candidats idéals pour préparer des émulsions huile-dans-eau exemptes de tensio-actifs émulsionnants.

Cependant, lorsque des émulsions huile-dans-eau sont préparées par la mise en oeuvre de tels terpolymères épaississants synthétiques en absence de tensio-actifs émulsionnants, on observe l'apparition d'amas dans lesdites émulsions huile-dans-eau exemptes de tensio-actifs émulsionnants lors de leur stockage au cours du temps. Il est donc nécessaire de développer de nouvelles émulsions huile-dans-eau, exemptes de tensio-actifs émulsionnants, qui ne présentent pas lors de leur stockage prolongé d'amas au stockage, mais qui conservent une viscosité élevée en présence de milieux riches en électrolytes et sur une large gamme de pH, ainsi que des propriétés sensorielles satisfaisantes, à savoir exempte de caractère collant et filant lors de leur préhension et après application sur la peau.

Les polysaccharides sont utilisés depuis de nombreuses années comme agents modificateurs de la texture et/ou de la rhéologie pour la préparation de compositions alimentaires, cosmétiques ou pharmaceutiques. Selon leur constitution chimique, ils peuvent être utilisés comme agents gélifiants et/ou comme agents épaississants. Par agent épaississant, on entend un composé chimique qui augmente la viscosité du milieu dans lequel il est introduit. Par agent gélifiant, on entend un composé qui transforme un milieu liquide en un état structuré, qui ne coule pas, par formation d'un réseau tridimensionnel au sein du liquide ; le gel étant considéré comme un état intermédiaire entre l'état liquide et l'état solide.

Les polysaccharides sont des polymères de saccharides. La définition IUPAC des saccharides désigne des oses, des composés d'oses proprement dits et leurs dérivés obtenus soit par réduction d'un groupement carbonyle, soit

par oxydation d'une ou plusieurs fonctions hydroxyles, soit par le remplacement d'une ou plusieurs fonctions hydroxyles par un atome d'hydrogène, par un groupement amine, une fonction phosphate, une fonction sulfate.

Les polysaccharides les plus couramment utilisés pour la préparation de compositions alimentaires, cosmétiques ou pharmaceutiques, sont majoritairement constitués d'oses, tels que le glucose, le galactose, le mannose ou de dérivés d'oses pour lesquels la fonction hydroxyle du carbone terminal a été oxydée en fonction carboxyle. On peut distinguer parmi les polysaccharides deux groupes distincts : les polysaccharides constitués uniquement d'oses (ou poly-oses) et les polysaccharides constitués de dérivés d'oses.

Parmi les polysaccharides composés uniquement d'oses, on peut distinguer les glucanes, qui sont des homopolymères de glucose très abondants dans la nature, les glucomannoglycanes, les xyloglycanes et les galactomannanes, qui sont des polymères dont la chaîne principale est constituée d'unités de D-mannose, reliées entre elles en $\beta$-1,4, et sur laquelle des unités de D-galactose sont greffées latéralement par des liaisons $\alpha$-1,6.

Les galactomannanes sont présents dans plusieurs espèces végétales, et plus particulièrement dans les espèces légumineuses dans lesquelles ils constituent l'albumen des graines. Selon leur origine végétale, le degré de substitution (DS) des unités de D-galactose sur la chaîne principale de D-mannose des galactomannanes, varie entre 0 et 1 :

- les galactomannanes provenant de la gomme de cassia présentent un degré de substitution (DS) d'environ 1/5, signifiant le greffage latéral d'une unité de D-galactose toutes les 5 unités de D-mannose présentes sur la chaîne principale du polysaccharide
- les galactomannanes provenant de la gomme de caroube présentent un degré de substitution (DS) d'environ 1/4, signifiant le greffage latéral d'une unité de D-galactose toutes les 4 unités de D-mannose présentes sur la chaîne principale du polysaccharide
- les galactomannanes provenant de la gomme de tara présentent un degré de substitution (DS) d'environ 1/3, signifiant le greffage latéral d'une unité de D-galactose toutes les 3 unités de D-mannose présentes sur la chaîne principale du polysaccharide
- les galactomannanes provenant de la gomme de guar présentent un degré de substitution (DS) d'environ 1/2, signifiant le greffage latéral d'une unité de D-galactose toutes les 2 unités de D-mannose présentes sur la chaîne principale du polysaccharide
- les galactomannanes provenant de la gomme de fenugrec présentent un degré de substitution (DS) d'environ 1/1, signifiant le greffage latéral d'une unité de D-galactose pour quasiment toutes les unités de D-mannose présentes sur la chaîne principale du polysaccharide.

En tant que sous-catégorie des polysaccharides, les galactomannanes ont déjà été associés à des épaississants synthétiques résultant de la polymérisation radicalaire de monomères tels que l'acide acrylique, les esters de l'acide acrylique, l'acide 2-acrylamido 2-méthyl propanesulfonique et/ou de ses sels, l'acrylamide, l'acrylate de (2-hydroxy éthyle).

Le brevet américain publié sous le numéro 4, 540, 510 décrit la préparation de gels aqueux mettant en oeuvre des homopolymères de l'acide 2-acrylamido 2-méthyl propanesulfonique et des galactomannanes, comme ceux issus de la gomme de guar et de la gomme de tara. Cependant, les gels aqueux qui sont obtenus et utilisés pour des applications dans le domaine de la fracturation des cavités souterraines, ou de la préparation de pâtes pigmentaires pour des impressions sur du textile, ou de la suspension de pigment dans des peintures, ou dans la préparation de formulations cosmétiques comprenant des alcools comme cosolvants, ne permettent pas d'atteindre des niveaux de viscosité suffisants pour permettre la préparation d'émulsions huile-dans-eau riches en électrolytes et stables au stockage.

La demande de brevet français publiée sous le numéro 2 940 111 décrit l'utilisation de compositions comprenant des polysaccharides, pouvant être associés à des gélifiants hydrophiles notamment choisis parmi des copolymères comportant l'acide 2-acrylamido-2-méthyl-propanesulfonique et l'acrylamide comme monomères constitutifs, ou des comportant l'acide 2-acrylamido 2-méthyl propanesulfonique et méthacrylates d'alkyles polyoxyéthylénés. Ces compositions sont destinées à des utilisations de maquillage, possédant la propriété de ne pas transférer sur les supports sur lesquelles elles sont mises en contact ainsi que la propriété consistant à résister à l'eau après application sur la peau. Cependant, les gélifiants hydrophiles décrits dans la demande de brevet Français publiée sous le numéro 2 940 111 sont connus pour ne pas permettre d'atteindre des niveaux élevés de viscosité en présence de milieux riches en électrolytes.

La demande de brevet américain publiée sous le numéro US 2011/0274629 A1 décrit des mélanges de galactomannanes et d'une gomme de xanthane modifiée, se caractérisant par une teneur en pyruvate inférieure à 0,5% massique, et leur utilisation comme agents épaississants pour préparer des compositions cosmétiques.

La demande de brevet européen publiée sous le numéro EP 0 152 095 A1 décrit des compositions comprenant des homopolymères de l'acide 2-acrylamido 2-méthyl propanesulfonique ou de l'acide 2-méthacrylamido 2-méthyl propanesulfonique, des galactomannanes, et leurs utilisations comme agents épaississants de milieux aqueux ou alcooliques, dans des applications de forage pétrolier, de préparation de pâtes d'impression pour textiles, et de préparation de

compositions cosmétiques comprenant des alcools.

La demande de brevet français publiée sous le numéro 2 466 273 décrit des émulsions cosmétiques dont la phase aqueuse est épaissie par gommes naturelles, plus particulièrement des galactomannanes et encore plus particulièrement la gomme de tara.

Les inventeurs ont donc cherché à développer de nouvelles émulsions huile-dans-eau, exemptes de tensioactifs émulsionnants dans leur système stabilisant, riches en sels, conservant une viscosité élevée et un aspect homogène après une période de stockage prolongée.

C'est pourquoi selon un premier aspect, l'invention a pour objet une composition ($C_1$) se présentant sous la forme d'une émulsion de type huile-dans-eau, caractérisée en ce qu'elle comprend pour 100% de sa masse :

- de 5% à 55% massique, plus particulièrement de 7% à 30% massique, et encore plus particulièrement de 10% à 20% massique d'une phase grasse ($P_1$) constituée d'au moins une huile et optionnellement d'au moins une cire,
- de 0,025% à 3,75% massique, plus particulièrement de 0,125% à 3% massique, et encore plus particulièrement de 0,125% à 2,25% massique d'au moins un polyélectrolyte anionique réticulé (PA) issu de la polymérisation de l'acide 2-méthyl 2-[(1-oxo 2-propènyl) amino] 1-propanesulfonique partiellement ou totalement salifié, avec au moins un monomère neutre choisi parmi l'acrylate de (2-hydroxy éthyle) et le N,N diméthylacrylamide, et au moins un monomère de formule (I) :

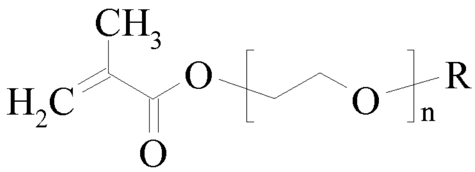

(I)

dans laquelle R représente un radical alkyle linéaire ou ramifié comportant de huit à vingt atomes de carbone et n représente un nombre supérieur ou égal à un et inférieur ou égal à vingt, en présence d'au moins un agent de réticulation,
- de 0,025% à 3,75% massique, plus particulièrement de 0,125% à 3% massique, et encore plus particulièrement de 0,125% à 2,25% massique d'au moins un galactomannane (GM) provenant de la gomme de tara et, présentant un degré de substitution (DS) d'environ 1/3,
- de 37,5% à 94,95% massique, plus particulièrement de 55% à 94,95% massique, et encore plus particulièrement de 65% à 94,95% massique d'une phase aqueuse ($P_2$) cosmétiquement acceptable, ladite phase aqueuse ($P_2$) comprenant pour 100% de sa masse de 1% à 15% massique d'au moins un sel (S) se présentant sous une forme dissoute,

ladite composition ($C_1$) étant en outre caractérisée en ce que le rapport massique entre le galactomannane (GM) et le polyélectrolyte anionique réticulé (PA) est supérieur ou égal à 1/3 et inférieur ou égal à 3/1, plus particulièrement supérieur ou égal à 1/2 et inférieur ou égal à 3/2, et encore plus particulièrement supérieur ou égal à 2/3 et inférieur ou égal à 1, et en ce que ledit polyélectrolyte anionique réticulé (PA) comporte pour 100% molaire de ses monomères constitutifs :
- De 20% molaire à 80% molaire d'unités monomériques issues dudit acide 2-méthyl 2-[(1-oxo 2-propènyl) amino] 1-propanesulfonique partiellement ou totalement salifiée ;
- De 15% molaire à 75% molaire d'unités monomériques issues dudit monomère neutre;
- De 0,5% à 5% molaire d'unités monomériques issues dudit monomère de formule (I) telle que définie précédemment.

Par huile, on désigne, dans la définition de la composition ($C_1$) objet de la présente invention, un composé et/ou un mélange de composés insoluble dans l'eau, et liquide à 25°C, comme celle choisie notamment parmi :

- Les huiles minérales telles que l'huile de paraffine, l'huile de vaseline, les isoparaffines ou les huiles blanches minérales ;
- Les huiles d'origine animale, telles que le squalène ou le squalane,
- Les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;

- Les huiles végétales éthoxylées ;
- Les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les poly(alpha-oléfine), les polyoléfines comme le poly(isobutane), les isoalcanes de synthèse comme l'isohexadécane, l'isododécane, les huiles perfluorées et
- Les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Par cire, on désigne, dans la définition de la composition ($C_1$) objet de la présente invention, notamment la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène ; les cires de silicone ; les cires végétales ; les alcools gras et les acides gras solides à température ambiante ; les glycérides solides à température ambiante.

Par galactomannane (GM) présentant un degré de substitution (DS) d'environ 1/3, on désigne, dans la définition de la composition ($C_1$) objet de la présente invention, un polysaccharide dont la chaîne principale est constituée d'unités de D-mannose, reliées entre elles en position $\beta$-1,4, et sur laquelle des unités de D-galactose sont greffées latéralement par des liaisons $\alpha$-1,6, de façon à ce que le greffage d'une unité de D-galactose s'observe en moyenne toutes les 3 unités de D-mannose présentes sur la chaîne principale dudit polysaccharide. Le galactomannane (GM) tel que défini précédemment provient plus particulièrement de la gomme de tara.

Par polyélectrolyte anionique réticulé (PA), on désigne, dans la définition de la composition ($C_1$) objet de la présente invention, un polyélectrolyte anionique réticulé non linéaire, se présentant à l'état de réseau tridimensionnel insoluble dans l'eau, mais gonflable à l'eau et conduisant à l'obtention d'un gel chimique.

Par partiellement salifié ou totalement salifié, on signifie dans la définition du polyélectrolyte anionique réticulé (PA) présent de la composition ($C_1$) telle que définie ci-dessus, que ledit acide 2-méthyl 2-[(1-oxo 2-propènyl) amino] 1-propanesulfonique est, partiellement ou totalement salifié, notamment sous forme de sel de métal alcalin, par exemple sous forme de sel de sodium ou de sel de potassium, ou sous forme de sel d'ammonium.

Dans la formule (I) telle que définie ci-dessus, par radical alkyle linéaire ou ramifié comportant de huit à vingt atomes de carbone, on désigne plus particulièrement pour R :

- ou bien un radical dérivé des alcools primaires linéaires tels que par exemple, le radical octyle, décyle, undécyle, dodécyle, tridécyle, tétradécyle, pentadécyle, hexadécyle, heptadécyle, octadécyle, nonadécyle ou eicosyle ;
- ou bien un radical dérivé des alcools de Guerbet, qui sont des 1-alcanols ramifiés répondant à la formule générale :

  $$CH_3\text{-}(CH_2)_p\text{-}CH[CH_3\text{-}(CH_2)_{p-2}]\text{-}CH_2OH,$$

  dans laquelle p représente un nombre entier compris entre 2 et 9, tels que, par exemple, les radicaux 2-éthyl hexyle, 2-propyl heptyle, 2-butyl octyle, 2-pentyl nonyle, 2-hexyl décyle ou 2-octyl dodécyle ;
- ou bien un radical dérivé des isoalcanols répondant à la formule générale :

  $$CH_3\text{-}CH(CH_3)\text{-}(CH_2)_m\text{-}CH_2OH,$$

  dans laquelle m représente un nombre entier compris entre 2 et 16, tels que, par exemple, les radicaux 4-méthyl pentyle, 5-méthyl hexyle, 6-méthyl heptyle, 15-méthyl pendadécyle ou 16-méthyl heptadécyle, soit les radicaux 2-hexyl octyle, 2-octyl décyle ou 2-hexyl dodécyle.

Selon un aspect particulier de la présente invention, celle-ci a pour objet une composition ($C_1$) telle que définie précédemment, pour laquelle dans la formule (I) telle que définie précédemment, R représente un radical alkyle comportant de 12 à 18 atomes de carbone.

Selon un autre aspect particulier, l'invention a pour objet une composition ($C_1$) telle que définie précédemment, pour laquelle dans la formule (I) telle que définie précédemment, n représente un nombre entier compris entre 3 et 20.

Selon un aspect encore plus particulier, l'invention a pour objet une composition ($C_1$) telle que définie précédemment, pour laquelle ledit monomère de formule (I) est le méthacrylate de lauryle tétraéthoxylé, le méthacrylate de

stéaryle eicosaéthoxylé, ou le méthacrylate de béhényle éthoxylé à 25 moles d'oxyde d'éthylène, et tout particulièrement le méthacrylate de lauryle tétraéthoxylé.

Selon un autre aspect particulier, l'invention a pour objet une composition ($C_1$) telle que définie précédemment, dans laquelle ledit polyélectrolyte anionique réticulé (PA) est réticulé avec un composé diéthylénique ou polyéthylènique dans la proportion molaire exprimée par rapport aux monomères mis en oeuvre, de 0,005% à 1%, plus particulièrement de 0,01% à 0,5% et tout particulièrement de 0,01% à 0,25%. L'agent de réticulation est plus particulièrement choisi parmi le diméthacrylate d'éthylèneglycol, le tétraallyloxyéthane, le diacrylate d'éthylèneglycol, le diallyl urée, le triallyl amine, le triméthylol propanetriacrylate ou le méthylène-bis(acrylamide) ou un mélange de ces composés.

Le polyélectrolyte anionique réticulé (PA) mis en oeuvre dans la composition ($C_1$) telle que définie précédemment et objet de la présente invention peut également comprendre divers additifs, tels que des agents complexants, des agents de transfert ou des agents limiteurs de chaîne.

Selon un aspect particulier, l'invention a pour objet une composition ($C_1$) telle que décrite précédemment dans laquelle ledit polyélectrolyte anionique réticulé (PA) est choisi parmi :

- Les terpolymères de l'acide 2-méthyl 2-[(1-oxo 2-propènyl) amino] 1-propanesulfonique partiellement salifié sous forme de sel d'ammonium, de l'acrylate de (2-hydroxy éthyle) et du méthacrylate de lauryle tétraéthoxylé, réticulé au triméthylol propanetriacrylate ;
- Les terpolymères de l'acide 2-méthyl 2-[(1-oxo 2-propènyl) amino] 1-propanesulfonique partiellement salifié sous forme de sel d'ammonium, de l'acrylate de (2-hydroxy éthyle) et du méthacrylate de stéaryle d'eicosaéthoxylé, réticulé au triméthylol propanetriacrylate ;
- Les terpolymères de l'acide 2-méthyl 2-[(1-oxo 2-propènyl) amino] 1-propanesulfonique partiellement salifié sous forme de d'ammonium, du N,N-diméthyl acrylamide et du méthacrylate de lauryle tétraéthoxylé, réticulé au triméthylol propanetriacrylate ; ou
- Les terpolymères de l'acide 2-méthyl 2-[(1-oxo 2-propènyl) amino] 1-propanesulfonique partiellement salifié sous forme de sel d'ammonium, du N,N-diméthyl acrylamide et du méthacrylate de stéaryle d'eicosaéthoxylé, réticulé au triméthylol propanetriacrylate.

Selon un aspect encore plus particulier, l'invention a pour objet une composition ($C_1$) telle que décrite précédemment dans laquelle ledit polyélectrolyte anionique réticulé (PA) est choisi parmi :

- Les terpolymères de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate ; ou
- Les terpolymères de l'acide 2-méthyl 2- [(1-oxo 2-propènyl) amino] 1-propanesulfonique partiellement salifié sous forme de sel d'ammonium, de l'acrylate de (2-hydroxy éthyle) et du méthacrylate de lauryle tétraéthoxylé, réticulé au triméthylol propanetriacrylate.

Dans la composition ($C_1$) selon l'invention, la combinaison in situ du polyélectrolyte anionique réticulé (PA) et du galactomannane (GM) présentant un degré de substitution (DS) d'environ 1/3, tels que définis précédemment et dans les proportions telles que définies précédemment, constitue le système stabilisant de ladite composition ($C_1$).

L'expression « cosmétiquement acceptable » utilisée dans la définition de la phase aqueuse ($P_2$) de la composition ($C_1$) objet de la présente invention, signifie selon la directive du Conseil de la Communauté Economique Européenne N°76/768/CEE du 27 juillet 1976 modifiée par la directive N°93/35/CEE du 14 juin 1993, que ladite phase aqueuse ($P_2$) comprend de l'eau et toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

Une phase aqueuse ($P_2$) cosmétiquement acceptable comprise dans la composition ($C_1$) objet de la présente invention contient de l'eau, et peut contenir classiquement un ou plusieurs solvants organiques hydrosolubles cosmétiquement acceptables, un mélange d'eau et d'un ou plusieurs solvants organiques cosmétiquement acceptables. Les solvants cosmétiquement acceptables peuvent plus particulièrement être choisis parmi les alcools polyhydriques comme par exemple le glycérol, le diglycérol, le triglycérol, les oligomères du glycérol, le xylitol, l'érythritol, le sorbitol, le 2-méthyl propanediol-1,3 ; les alcools polyhydriques alcoxylés ; les glycols, comme par exemple le butylèneglycol, l'hexylèneglycol, le caprylylglycol ou 1,2-octanediol ou 1,2-pentanediol, le pentylèneglycol, le monopropylèneglycol, le dipropylèneglycol, l'isoprèneglycol, le butyldiglycol, les polyéthylènes glycols dont le poids moléculaire est compris entre $200g.mol^{-1}$ et $8000g.mol^{-1}$ ; ou les alcools hydrosolubles comme par exemple l'éthanol, l'isopropanol ou le butanol.

Dans la composition ($C_1$) telle que définie précédemment, par sel (S) on désigne un composé hétéropolaire dont le réseau cristallin comprend la participation d'au moins un type de cation différent de l'ion hydrogène et d'au moins

un type d'anion différent de l'ion hydroxyde.

Selon un aspect particulier, le sel (S) se présentant sous une forme dissoute dans la phase aqueuse ($P_2$) de la composition ($C_1$) objet de la présente invention, est sélectionné parmi les sels inorganiques ou parmi les sels organiques.

Selon un aspect plus particulier, le sel (S) est particulièrement sélectionné parmi les sels inorganiques et il est notamment constitué par un cation qui est l'ion ammonium ou un cation métallique et par un anion sélectionné parmi les éléments du groupe constitué par les halogénures, les carbonates, les bicarbonates, les phosphates, les nitrates, les borates et les sulfates.

Selon un aspect plus particulier, l'invention a pour objet une composition ($C_1$) telle que définie précédemment caractérisée en ce que le sel (S) est un sel inorganique dont le cation métallique est un cation monovalent ou multivalent choisi parmi les éléments du groupe constitué par les cations du sodium, du potassium, du lithium, du calcium, du magnésium, du zinc, du manganèse, du fer, du cuivre, du cobalt, de l'argent, de l'or, de l'aluminium, du barium, du bismuth, du sélénium, du zirconium, du strontium et de l'étain.

Selon un aspect encore plus particulier, l'invention a pour objet une composition ($C_1$) telle que définie précédemment caractérisée en ce que le sel (S) est un sel inorganique choisi parmi les éléments du groupe constitué par le chlorure de sodium, le chlorure de calcium, le chlorure de magnésium, le sulfate de calcium, le sulfate d'ammonium, le carbonate de calcium, le sulfate de zinc, le sulfate de magnésium, le borate de sodium.

Selon un autre aspect particulier, le sel (S) est particulièrement sélectionné parmi les sels organiques.

Selon un aspect particulier, l'invention a pour objet une composition ($C_1$) telle que définie précédemment caractérisée en ce que le sel (S) est un sel organique constitué par un cation qui est l'ion ammonium ou un cation métallique et par un anion organique qui est un composé organique possédant au moins une fonction acide carboxylique sous forme carboxylate ou au moins une fonction acide sulfonique sous forme sulfonate ou au moins une fonction sulphate.

Selon cet aspect particulier, l'invention a pour objet une composition ($C_1$) telle que définie précédemment caractérisée en ce que le sel (S) est un sel organique constitué par un cation métallique monovalent ou multivalent plus particulièrement choisi parmi les éléments du groupe constitué par les cations du sodium, du potassium, du lithium, du calcium, du magnésium, du zinc, du manganèse, du fer, du cuivre, du cobalt de l'argent, de l'or, de l'aluminium, du barium, du bismuth, du sélénium, du zirconium, du strontium et de l'étain. Selon cet aspect particulier, le sel (S) est un sel organique constitué par le cation choisi parmi les éléments groupe constitué par les cations du sodium, du calcium, du magnésium, du zinc et du manganèse, et encore plus particulièrement le sel (S) est un sel organique constitué par le cation du sodium.

Selon un aspect particulier, l'invention a pour objet une composition ($C_1$) telle que définie précédemment caractérisée en ce que le sel (S) est un sel organique constitué par un cation qui est l'ion ammonium ou un cation métallique tel que décrit précédemment, et par un anion organique qui est un composé organique possédant au moins une fonction acide carboxylique sous forme carboxylate choisi parmi les éléments du groupe constitué par l'acide glycolique, l'acide citrique, l'acide tartrique, l'acide salicylique, l'acide lactique, l'acide mandélique, l'acide ascorbique, l'acide pyruvique, l'acide fumarique, l'acide rétinoïque, l'acide benzoïque, l'acide kojique, l'acide malique, l'acide gluconique, l'acide galacturonique, l'acide proprionique, l'acide heptanoïque, l'acide 4-amino benzoïque, l'acide cinnamique, l'acide benzalmalonique, l'acide aspartique et l'acide glutamique.

Selon un aspect encore plus particulier, l'invention a pour objet une composition ($C_1$) telle que définie précédemment caractérisée en ce que le sel (S) est un sel organique sélectionné parmi les éléments du groupe constitué par le glycolate de sodium, le citrate de sodium, le salicylate de sodium, le lactate de sodium, le gluconate de sodium, le gluconate de zinc, le gluconate de manganèse, le gluconate de cuivre et l'aspartate de magnésium.

Selon un autre aspect particulier, l'invention a pour objet une composition ($C_1$) telle que définie précédemment caractérisée en ce que le sel (S) est un sel organique constitué par un cation qui est l'ion ammonium ou un cation métallique tel que décrit précédemment, et par un anion organique qui est un composé organique possédant au moins une fonction acide sulfonique sous forme sulfonate choisi parmi les éléments du groupe constitué par l'acide 2-phénylbenzimidazole-5-sulphonique, les acides sulphoniques dérivés des benzophénones, comme par exemple l'acide 4-hydroxy-2-methoxy-5-(oxophénylméthyl)benzène sulphonique (ledit acide étant enregistré sous l'appellation Benzophénone-4), les acides sulphoniques dérivés du 3-benzylidene camphre comme par exemple l'acide 4-(2-oxo-3-bornylidenemethyl) benzènesulphonique, l'acide 2-méthyl-5 (2-oxo-3-bornylideneméthyl) benzènesulphonique.

Selon un aspect encore plus particulier, l'invention a pour objet une composition ($C_1$) telle que définie précédemment caractérisée en ce que le sel (S) est un sel organique sélectionné parmi les éléments du groupe constitué par le 2-phénylbenzimidazole-5-sulphonate de sodium et le 4-hydroxy 2-méthoxy 5-(oxo-phénylméthyl)benzènesulphonate de sodium. L'acide 2-phénylbenzimidazole-5-sulphonique est commercialisé notamment sous le nom de marque EUSOLEX™232 par la société Merck. Le 4-hydroxy-2-methoxy-5-(oxo-phénylméthyl)benzène sulphonate de sodium est enregistré sous l'appellation benzophénone-5.

De façon générale, la composition ($C_1$) objet de la présente invention comporte, en plus de ladite phase grasse

($P_1$), du système stabilisant, composé de la combinaison in situ du polyélectrolyte anionique réticulé (PA) et du galactomannane (GM) tels que définis précédemment, et de ladite phase aqueuse cosmétiquement acceptable ($P_2$) des adjuvants et ou des additifs habituellement mis en oeuvre dans le domaine des formulations cosmétiques, dermocosmétiques, pharmaceutiques ou dermo-pharmaceutiques.

Parmi les adjuvants susceptibles d'être présents dans ladite composition ($C_1$), il y a les composés filmogènes, les agents hydrotropes, les agents plastifiants, les agents opacificants, les agents nacrants, les agents surgraissants, les agents séquestrants, les agents chélatants, les agents tensioactifs détergents non-ioniques, les agents antioxydants, les parfums, les agents conservateurs, les agents conditionneurs, les agents blanchissants destinés à la décoloration des poils et de la peau, les principes actifs destinés à apporter une action traitante vis à vis de la peau ou des cheveux, les charges minérales ou les pigments, les particules procurant un effet visuel ou destinées à l'encapsulation d'actifs, les particules exfoliantes, les agents de texture, les azurants optiques, des répulsifs pour les insectes.

Comme agents opacifiants et/ou nacrants il y a par exemple les palmitate, les stéarates ou les hydroxystéarates de sodium ou de magnésium, les monostéarate ou distéarate d'éthylèneglycol ou de polyéthylèneglycol, les alcools gras, les homopolymères et copolymères de styrène tels que le styrèneacrylate copolymère commercialisé sous l'appellation MONTOPOL™OP1 par la société SEPPIC.

Parmi les agents de texture que l'on peut associer à ladite composition ($C_1$), on peut citer des dérives N-acyles d'acides amines, comme la lauroyl lysine commercialisée sous l'appellation AMINOHOPE™LL par la société AJINOMOTO, l'octenyl starch succinate commercialise sous l'appellation DRYFLO™ par la société NATIONAL STARCH, le myristyl polyglucoside commercialisé par SEPPIC sous l'appellation MONTANOV™ 14, les fibres de cellulose, les fibres de coton, les fibres de chitosane, le talc, la sericite, le mica.

Parmi les principes actifs que l'on peut associer à ladite composition ($C_1$), on peut citer par exemple les vitamines et leurs dérives, notamment leurs esters, tels que le rétinol (vitamine A) et ses esters (palmitate de rétinyle par exemple), l'acide ascorbique (vitamine C) et ses esters, les dérives de sucre de l'acide ascorbique (comme par exemple l'ascorbyl glucoside), le tocophérol (vitamine E) et ses esters (comme par exemple l'acétate de tocophérol), les vitamine B3 ou B10 (niacinamide et ses dérivés); les composés montrant une action éclaircissante ou dépigmentante de la peau comme par exemple le SEPIWHITE™MSH, l'arbutine, l'acide kojique, l'hydroquinone, le VEGEWHITE™, la GATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™; les composés montrant une action apaisante comme le SEPICALM™ S, l'allantoïne et le bisabolol ; les agents anti-inflammatoires ; les composés montrant une action hydratante comme par exemple l'urée, les hydroxyurées, le glycérol, les polyglycérols, l'AQUAXYL™, le glycérolglucoside ; les extraits de polyphénols comme par exemple les extraits de raisin, les extraits de pin, les extraits de vin, les extraits d'olives ; les composé montrant une action amincissante ou lipolytique comme la caféine ou ses dérivés, l'ADIPOSLIM™, l'ADIPOLESS™ ; les protéines N-acylées ; les peptides N-acylés comme par exemple le MATRIXIL™ ; les acides aminés N-acylés ; les hydrolysâts partiels de protéines N-acylés ; les acides aminés ; les peptides ; les hydrolysâts totaux de protéines ; les extraits de soja, par exemple la Raffermine™ ; les extraits de blé par exemple la TENSINE™ ou la GLIADINE™ ; les extraits végétaux, tels que les extraits végétaux riches en tanins, les extraits végétaux riches en isoflavones ou les extraits végétaux riches en terpènes ; les extraits d'algues d'eau douce ou marines ; les extraits marins en général comme les coraux ; les cires essentielles ; les extraits bactériens ; les céramides ; les phospholipides ; les composés montrant une action antimicrobienne ou une action purifiante, comme par exemple le LIPACIDE™ C8G, le LIPACIDE™ UG, le SEPICONTROL™ A5 ; l'OCTOPIROX™ ou le SENSIVA™ SC50 ; les composés montrant une propriété énergisante ou stimulante comme le Physiogényl™, le panthénol et ses dérivés comme le SEPICAP™ MP ; les actifs anti-age comme le SEPILIFT™ DPHP, le LIPACIDE™ PVB, le SEPIVINOL™, le SEPIVITAL™, le MANOLIVA™, le PHYTO-AGE™, le TIMECODE™ ; le SURVICODE™ ; les actifs anti-photo vieillissement ; les actifs protecteurs de l'intégrité de la jonction dermo-épidermique ; les actifs augmentant la synthèse des composants de la matrice extracellulaire comme par exemple le collagène, les élastines, les glycosaminoglycanes ; les actifs agissant favorablement sur la communication cellulaire chimique comme les cytokines ou physiques comme les intégrines ; les actifs créant une sensation de « chauffe » sur la peau comme les activateurs de la microcirculation cutanée (comme par exemple les dérivés de l'acide nicotinique) ou des produits créant une sensation de « fraîcheur » sur la peau (comme par exemple le menthol et des dérivés) ; les actifs améliorant la microcirculation cutanée, par exemple les veinotoniques ; les actifs drainants ; les actifs à visée décongestionnante comme par exemple les extraits de ginko biloba, de lierre, de marron d'inde, de bambou, de ruscus, de petit houx, de centalla asiatica, de fucus, de romarin, de saule.

Parmi les principes actifs que l'on peut associer à ladite composition ($C_1$), on peut citer plus particulièrement les agents de bronzage ou de brunissement de la peau, comme par exemple la dihydroxyacétone, l'isatinne, l'alloxane, la ninhydrine, le glycéraldéhyde, l'aldéhyde mésotartrique, la glutaraldéhyde, l'érythrulose.

Parmi les agents tensioactifs détergents non ioniques que l'on peut associer à ladite composition ($C_1$), on peut citer les dérivés éthoxylés d'alcools gras comportant de 8 à 12 atomes de carbone, les dérivés éthoxylés d'acides gras comportant de 8 à 12 atomes de carbone, les dérivés éthoxylés d'esters gras comportant de 8 à 12 atomes de carbone,

les dérivés éthoxylés de monoglycérides comportant de 8 à 12 atomes de carbone,les alkylpolyglycosides de formule (II) :

$$R_2\text{-O-(S)}_y\text{-H} \qquad \text{(II)}$$

dans laquelle y représente un nombre décimal compris entre 1 et 5, S représente le reste d'un sucre réducteur et $R_2$ représente un radical alkyle linéaire ou ramifié, saturé ou insaturé, ayant de 5 à 16 atomes de carbone, de préférence de 8 à 14 atomes de carbone, ou un mélange de composés de formule (II).

Les agents tensioactifs détergents non ioniques que l'on peut associer à ladite composition ($C_1$), sont plus particulièrement choisi parmi les éléments du groupe constitué par le caprylyl capryl glucosides, commercialisé notamment sous le nom de marque ORAMIX™CG 110 par la société SEPPIC, le décylglucoside, commercialisé notamment sous le nom de marque ORAMIX™NS 10 par la société SEPPIC.

Parmi les pigments que l'on peut associer à ladite composition ($C_1$), on peut citer le dioxyde de titane, les oxydes de fer marrons, les oxydes de fer jaune, les oxydes de fer noir, ou les oxydes de fer rouges ou encore les pigments nacrés blancs ou colorés tels que les Mica-Titane.

Parmi les filtres solaires que l'on peut associer à ladite composition ($C_1$), on peut citer tous ceux figurant dans la directive cosmétique 76/768/CEE modifiée annexe VII, comme par exemple l'oxyde de titane, l'oxyde de zinc, les esters de l'acide cinnamique comme par exemple le 4-méthoxy cinnamate de 2-éthyl hexyle, le 4-méthoxy cinnamate d'isopentyle, les dérivés non ioniques de benzophénone, les esters de l'acide 4-amino benzoïque comme par exemple le 4-(diméthylamino) benzoate de 2-éthyl hexyle, le 4-(diméthylamino) benzoate d'amyle.

Selon un autre aspect particulier, l'invention a pour objet une composition ($C_1$) telle que définie précédemment, caractérisée en ce que sa viscosité dynamique mesurée à une température de 20°C, au moyen d'un viscosimètre de type Brookfield est supérieure ou égale à 30.000mPa.s et inférieure ou égale à 200.000mPa.s, plus particulièrement supérieure ou égale à 40.000 mPa.s et inférieure ou égale à 130.000mPa.s, et encore plus particulièrement supérieure ou égale à 50.000mPa.s et inférieure ou égale à 130.000mPa.s.

Lorsque la viscosité dynamique de la composition ($C_1$) est inférieure ou égale à environ 100.000mPa.s à une température de 20°C, ladite viscosité dynamique est mesurée au moyen d'un viscosimètre de type Brookfield LVT à une vitesse de 6 tours par minutes.

Lorsque la viscosité dynamique de la composition ($C_1$) est supérieure à environ 100.000mPa.s à une température de 20°C, ladite viscosité dynamique est mesurée au moyen d'un viscosimètre de type Brookfield RVT à une vitesse de 5 tours par minute.

Selon un aspect particulier, l'invention a pour objet une composition ($C_1$) telle que définie précédemment, caractérisée en ce que la conductivité de ladite composition ($C_1$), mesurée à une température de 20°C au moyen d'un conductimètre de marque LF 196 de la société WTW muni d'une électrode Tétracon 96, est supérieure ou égale à 15 millisiemens.$cm^{-1}$ (mS.$cm^{-1}$) et inférieure ou égale à 200 mS.$cm^{-1}$, plus particulièrement supérieure ou égale à 15 mS.$cm^{-1}$ et inférieure ou égale à 150 mS.$cm^{-1}$.

Ladite composition ($C_1$) se présente notamment sous la forme d'une émulsion ou d'une microémulsion à phase continue aqueuse.

Lorsque ladite composition ($C_1$) possède des caractéristiques de fluidité appropriées, elle peut aussi servir pour l'imprégnation de supports constitués de fibres textiles synthétiques ou naturelles, tissées ou non tissées, ou de papiers, pour constituer des articles, comme par exemple des lingettes, destinés au soin, à la protection ou au nettoyage de la peau, du cuir chevelu ou des cheveux, ou comme par exemple des papiers a usage sanitaire ou domestique.

Ladite composition ($C_1$), peut être mise en oeuvre par application sur la peau, les muqueuses, les cheveux ou le cuir chevelu, qu'il s'agisse d'une application directe dans le cas d'une composition cosmétique, dermocosmétique, dermopharmaceutique ou pharmaceutique, ou d'une application indirecte dans le cas d'un produit de soin, de protection, de nettoyage du corps se présentant sous la forme d'un article en textile, comme par exemple une lingette, ou en papier, comme par exemple un papier à usage sanitaire, destines a être en contact avec la peau, les cheveux ou le cuir chevelu.

La composition ($C_1$) telle que définie ci-dessus et objet de la présente invention, est stable dans le temps après une période de stockage d'au moins un mois à 20°C et conserve un aspect homogène, ne montrant pas l'apparition de grumeaux ou d'amas, à l'issue de cette même période de stockage dans les mêmes conditions expérimentales, sans qu'il soit nécessaire d'incorporer dans ladite composition ($C_1$) des tensioactifs émulsionnants.

Selon un aspect particulier, la présente invention a pour objet une composition ($C_1$) comprenant pour 100% de sa masse une quantité de 0% massique d'agents tensioactifs émulsionnants (EM) choisi parmi les éléments du groupe ($G_1$) constitué par :

- les acides gras comportant de 14 à 22 atomes de carbone,
- les acides gras éthoxylés comportant de 14 à 22 atomes de carbone,
- les esters d'acide gras comportant de 14 à 22 atomes de carbone et de sorbitol,
- les esters d'acide gras comportant de 14 à 22 atomes de carbone et de polyglycérol,

- les alcools gras comportant de 14 à 22 atomes de carbone éthoxylés,
- les esters d'acide gras comportant de 14 à 22 atomes de carbone et de sucrose,
- les alkylpolyglycosides de formule (II) :

$$R_3\text{-O-(S)}_z\text{-H} \qquad \text{(III)}$$

dans laquelle z représente un nombre décimal compris entre 1 et 5, S représente le reste d'un sucre réducteur et $R_3$ représente un radical alkyle linéaire ou ramifié, saturé ou insaturé, ayant de 14 à 22 atomes de carbone, de préférence de 16 à 22 atomes de carbone, ou un mélange de composés de formule (III).

Dans la définition de la formule (III) telle que définie précédemment, z est un nombre décimal qui représente le degré moyen de polymérisation du reste S. Lorsque z est un nombre entier, $(S)_z$ est le reste polymérique de rang z du reste S. Lorsque z est un nombre décimal, la formule (III) représente un mélange de composés : $a_1$ $R_3$-O-S-H + $a_2$ $R_3$-O-$(S)_2$-H + $a_3$ $R_3$-O-$(S)_3$-H + ... + $a_q$ $R_3$-O-$(S)_q$-H avec q représentant un nombre entier compris entre 1 et 10 et dans les proportions molaires $a_1$, $a_2$, $a_3$,... $a_q$ telles que :

$$\sum_{q=1}^{q=10} a_q = 1 \; ; \; a_1 > 0$$

Dans la formule (III) telle que définie ci-dessus, z est compris entre 1,05 et 5,0 et plus particulièrement entre 1,05 et 2.

Dans la formule (III) telle que définie ci-dessus, $R_3$ représente par exemple le radical n-tétradécyle, le radical n-hexadécyle, le radical n-octadécyle, le radical n-eicosyle, le radical n-dodécosyle.

Par sucre réducteur, on désigne, dans la définition de la formule (III), les dérivés saccharidiques qui ne présentent pas dans leurs structures de liaison glycosidique établie entre un carbone anomérique et l'oxygène d'un groupement acétal tels qu'ils sont définis dans l'ouvrage de référence « Biochemistry », Daniel Voet/Judith G. Voet, p. 250, John Wyley & Sons, 1990. La structure oligomérique $(S)_z$, peut se présenter sous toute forme d'isomérie, qu'il s'agisse d'isomérie optique, d'isomérie géométrique ou d'isomérie de position ; elle peut aussi représenter un mélange d'isomères.

Dans la formule (III) telle que définie ci-dessus, le groupe $R_3$-O- est lié à S par le carbone anomérique du reste saccharide, de manière à former une fonction acétal.

Dans la formule (III) telle que définie ci-dessus, S représente le reste d'un sucre réducteur choisi parmi le glucose, le xylose ou l'arabinose.

Selon un autre aspect particulier, la présente invention a pour objet une composition ($C_1$) comprenant pour 100% de sa masse de 0,1% à 10% massique, plus particulièrement de 0,1% à 5% massique, et encore plus particulièrement de 0,5% à 3% massique d'au moins un agent tensioactif émulsionnant (EM) choisi parmi les éléments du groupe ($G_1$) tel que défini ci-dessus.

Selon cet autre aspect particulier, le ratio massique entre la somme de la quantité massique de polyélectrolyte anionique (PA) et la quantité massique du galactomannane (GM), tels que définis précédemment, et la quantité massique de l'agent émulsionnant (EM) est supérieur ou égal à 1,0, plus particulièrement supérieur ou égal à 5,0, et encore plus particulièrement supérieur ou égal à 10,0.

Selon un autre aspect, la présente invention a pour objet un procédé de préparation de la composition ($C_1$) telle que définie précédemment, comprenant :

Au moins une étape a) de préparation d'une phase ($P'_1$) par mélange du polyélectrolyte anionique réticulé (PA) et du galactomannane (GM) dans la phase grasse ($P_1$) ; et
Au moins une étape b) d'émulsification de ladite phase ($P'_1$) obtenue à l'issue de l'étape a) avec la phase aqueuse ($P_2$) cosmétiquement acceptable.

Dans le procédé objet de l'invention, la phase grasse ($P_1$) comprend une ou plusieurs huiles et/ou une ou plusieurs cires telles que définies précédemment.

Dans le cas où la phase grasse ($P_1$) n'est pas constituée par une seule huile ou par une seule cire, la phase

grasse ($P_1$) est préparée par mélange des ingrédients la constituant à une température typiquement comprise entre 20°C et 85°C, et encore plus particulièrement à une température comprise entre 20°C et 60°C, et par le moyen de tout dispositif de mélange connu de l'homme du métier, comme par exemple par le moyen d'un dispositif d'agitation mécanique équipé d'un mobile de type « ancre », à des vitesses d'agitation comprises entre 50 tours par minute et 500 tours par minutes, plus particulièrement entre 50 tours par minute et 300 tours par minutes.

Dans le procédé objet de l'invention tel que décrit ci-dessus, l'étape a) de préparation d'une phase ($P'_1$) par mélange du polyélectrolyte anionique réticulé (PA) et du galactomannane (GM) dans la phase grasse ($P_1$) peut être avantageusement mise en oeuvre à une température inférieure ou égale à 85°C et supérieure ou égale à 20°C, plus particulièrement à une température inférieure ou égale à 60°C et supérieure ou égale à 20°C.

Dans le procédé objet de l'invention tel que décrit ci-dessus, l'étape a) de préparation d'une phase ($P'_1$) par mélange du polyélectrolyte anionique réticulé (PA) et du galactomannane (GM) dans la phase grasse ($P_1$) peut être réalisée par le moyen de tout dispositif de mélange connu de l'homme du métier, comme par exemple le moyen d'un dispositif d'agitation mécanique équipé d'un mobile de type « ancre », à des vitesses d'agitation comprises entre 50 tours par minute et 500 tours par minutes, plus particulièrement entre 50 tours par minute et 300 tours par minutes, et comme par exemple par le moyen d'un dispositif d'agitation de type rotor-stator à des vitesses d'agitation comprises entre 100 tours par minute et 10 000 tours par minutes, plus particulièrement entre 500 tours par minutes et 4000 tours par minutes.

Dans le procédé objet de l'invention, l'étape b) d'émulsification de la phase ($P'_1$) obtenue à l'issue de l'étape a) avec la phase aqueuse ($P_2$).peut être avantageusement mise en oeuvre à une température comprise entre 20°C et 90°C, plus particulièrement à une température comprise entre 20°C et 85°C, et encore plus particulièrement à une température comprise entre 20°C et 60°C.

Dans le procédé objet de l'invention, l'étape b) d'émulsification de la phase ($P'_1$) obtenue à l'issue de l'étape a) avec la phase aqueuse ($P_2$) peut être réalisée par le moyen de tout dispositif de mélange connu de l'homme du métier, comme par exemple le moyen d'un dispositif d'agitation mécanique équipé d'un mobile de type « ancre », à des vitesses d'agitation comprises entre 50 tours par minute et 500 tours par minute, plus particulièrement entre 50 tours par minute et 300 tours par minute, et comme par exemple par le moyen d'un dispositif d'agitation de type rotor-stator a des vitesses d'agitation comprises entre 100 tours par minute et 10.000 tours par minute, plus particulièrement entre 500 tours par minute et 4.000 tours par minute.

Dans le procédé objet de l'invention tel que décrit ci-dessus, la phase aqueuse cosmétiquement acceptable ($P_2$) comprend de l'eau, et éventuellement un ou plusieurs solvants organiques cosmétiquement acceptables tels que décrits précédemment, et de 1% à 25% massique, pour 100% de la masse de ladite phase aqueuse ($P_2$) cosmétiquement acceptable d'au moins un sel (S) se présentant sous une forme dissoute et tel que défini précédemment.

La phase aqueuse ($P_2$) cosmétiquement acceptable est préparée par mélange de l'eau, et éventuellement d'un ou plusieurs solvants organiques cosmétiquement acceptable, avec au moins un sel (S) tel que décrit précédemment, à une température comprise entre 20°C et 85°C, et encore plus particulièrement à une température comprise entre 20°C et 60°C, et par le moyen de tout dispositif de mélange connu de l'homme du métier, comme par exemple par le moyen d'un dispositif d'agitation mécanique équipé d'un mobile de type « ancre », à des vitesses d'agitation comprises entre 50 tours par minute et 500 tours par minutes, plus particulièrement 20 entre 50 tours par minute et 300 tours par minutes.

Selon un autre aspect, la présente invention a pour objet l'utilisation cosmétique de la composition ($C_1$) telle que définie précédemment pour le nettoyage, pour la protection et/ou pour le soin de la peau, des cheveux, du cuir chevelu ou des muqueuses.

Dans le cadre de la présente invention, par « utilisation cosmétique » on désigne les utilisations de la composition ($C_1$) destinées à améliorer et/ou préserver l'aspect esthétique extérieur de la peau, des cheveux, du cuir chevelu ou des muqueuses.

Selon un aspect plus particulier, ladite composition ($C_1$) peut être utilisée pour le nettoyage de la peau, des muqueuses, des cheveux ou du cuir chevelu, et plus particulièrement peut être utilisée comme gel pour le bain ou pour la douche, comme shampooing. Dans cette utilisation particulière, elle comprend en outre au moins un tensioactif détergent non ionique tel que décrit précédemment.

Selon un autre aspect plus particulier, ladite composition ($C_1$), peut être utilisée pour le soin ou pour la protection de la peau, comme par exemple comme crème, comme lait ou comme lotion pour le soin ou pour la protection du visage, des mains et du corps.

Selon cet aspect particulier, ladite composition ($C_1$), peut aussi être utilisée plus particulièrement comme produit protecteur de la peau contre les rayons du soleil, comme produit de maquillage de la peau, comme produit protecteur de la peau contre le vieillissement cutané, comme produit hydratant de la peau, comme produit de traitement cosmétique de l'acné et/ou des points noirs et/ou des comédons.

Le rapport expérimental suivant illustre l'invention sans toutefois la limiter.

**1-1 Préparation d'un terpolymère de 2-méthyl 2-[(1-oxo 2-propènyl) amino] 1-propanesulfonate d'ammonium, de N,N-diméthyl acrylamide et de méthacrylate de lauryle tétraéthoxylé [$AMPSNH_4$/DMAM/MAL(4OE) 77,4/19,2/3,4 molaire], réticulé au propanetriacrylate de triméthylol (TMPTA)** [Exemple selon l'invention].

On charge dans un réacteur maintenu à 25°C sous agitation, 592g d'une solution aqueuse à 15% massique de 2-méthyl 2-[(1-oxo 2-propènyl) amino] 1-propanesulfonate d'ammonium ($AMPSNH_4$) dans un mélange tert-butanol/eau (97,5/2,5 en volume), 10,1g de N,N-diméthyl acrylamide (DMAM), 4,2g de méthacrylate de lauryle tétraéthoxylé [MAL(4OE)] et 0,75g de TMPTA.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : "Polyélectrolyte $PA_1$".

**1-2 : Préparation d'un terpolymère de 2-méthyl 2-[(1-oxo 2-propènyl) amino] 1-propanesulfonate d'ammonium, d'acrylate de (2-hydroxy éthyle) et de méthacrylate de lauryle tétraéthoxylé [$AMPSNH_4$/HEA/MAL(4OE) 77,4/19,2/3,4 molaire], réticulé au TMPTA** [Exemple selon l'invention].

En mettant en oeuvre les conditions opératoires du procédé décrit au paragraphe 1.1 précédent, on charge dans un réacteur maintenu à 25°C sous agitation, la quantité massique nécessaire d'une solution aqueuse à 15% massique d'$AMPSNH_4$ dans un mélange tert-butanol/eau (97,5/2,5 en volume) de façon à introduire 77,4 équivalents molaires d'$AMPSNH_4$, la quantité massique nécessaire d'acrylate de (2-hydroxy éthyle) (HEA) de façon à introduire 19,2 équivalents molaires d'HEA, la quantité massique nécessaire de [MAL(4OE)] de façon à introduire 3,4 équivalents molaires de [MAL(4OE)], et la quantité massique nécessaire de TMPTA de façon à obtenir la même proportion molaire en TMPTA qu'au paragraphe 1-1.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : "Polyélectrolyte $PA_2$".

**1-3 : Préparation d'un copolymère de 2-méthyl 2-[(1-oxo 2-propènyl) amino] 1-propanesulfonate d'ammonium et de méthacrylate de lauryle tétraéthoxylé [AMPS/MAL(4OE) 95/5 molaire], réticulé au TMPTA** [Exemple comparatif].

En mettant en oeuvre les conditions opératoires du procédé décrit au paragraphe 1-1 précédent, on charge dans un réacteur maintenu à 25°C sous agitation, la quantité massique nécessaire d'une solution aqueuse à 15% massique d'$AMPSNH_4$ dans un mélange tert-butanol/eau (97,5/2,5 en volume) de façon à introduire 95 équivalents molaires d'$AMPSNH_4$, la quantité massique nécessaire de [MAL(4OE)] de façon à introduire 5 équivalents molaires de [MAL(4OE)], et la quantité massique nécessaire de TMPTA de façon à obtenir la même proportion molaire en TMPTA qu'au paragraphe 1-1..

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : "Polyélectrolyte $PA_3$".

**1-4 : Préparation d'un copolymère de 2-méthyl 2-[(1-oxo 2-propènyl) amino] 1-propanesulfonate d'ammonium et d'acrylate de (2-hydroxy éthyle) [AMPS/HEA 90/10 molaire], réticulé au TMPTA** [Exemple comparatif].

En mettant en oeuvre les conditions opératoires du procédé décrit au paragraphe 1-1 précédent, on charge dans un réacteur maintenu à 25°C sous agitation, la quantité massique nécessaire d'une solution aqueuse à 15% massique d'$AMPSNH_4$ dans un mélange tert-butanol/eau (97,5/2,5 en volume) de façon à introduire 90 équivalents molaires d'$AMPSNH_4$, la quantité massique nécessaire d'HEA de façon à introduire 10 équivalents molaires d'HEA, et la quantité massique nécessaire de TMPTA de façon à obtenir la même proportion molaire en TMPTA qu'au paragraphe 1-1.

Après un temps suffisant pour atteindre une bonne homogénéisation de la solution, celle-ci est désoxygénée par barbotage d'azote chauffée à 70°C. 0,42g de peroxyde de dilauroyle sont alors ajoutés et le milieu réactionnel est

ensuite maintenu pendant environ 60 minutes à 70°C puis 2 heures à 80°C.

Après refroidissement, la poudre qui s'est formée en cours de polymérisation, est filtrée et séchée pour obtenir le produit désiré, dénommé par la suite : "Polyélectrolyte $PA_4$".

**2-1 : Préparation d'émulsions huile-dans-eau selon l'invention.**

On prépare treize émulsions huile-dans-eau selon l'invention, notées ($E_1$) à ($E_{13}$), dont les proportions massiques en leurs constituants sont consignées dans le tableau 1 ci-dessous, en mettant en oeuvre le procédé suivant :

- Dans un premier bécher, on disperse progressivement et successivement à une température de 20°C, l'un des polyélectrolytes $PA_1$ ou $PA_2$ et la gomme de Tara dans une phase grasse sous agitation mécanique à 80 tours par minute ;
- On prépare dans un second bécher, à une température de 20°C, la phase aqueuse comprenant l'eau et la quantité massique de sel souhaitée ;
- Le contenu du premier bécher est progressivement versé dans le second bécher à une température de 20°C, sous agitation mécanique au moyen d'une défloculeuse à 1.200 tours par minute ;
- Le mélange obtenu est maintenu sous agitation pendant 10 minutes, puis vidangé pour obtenir les émulsions huile-dans-eau ($E_1$) à ($E_{13}$).

| **Emulsion** | **($E_1$)** | **($E_2$)** | **($E_3$)** | **($E_4$)** | **($E_5$)** | **($E_6$)** |
|---|---|---|---|---|---|---|
| **Phase grasse :** | | | | | | |
| Triglycérides C8-C10 | 15% | 15% | 15% | 15% | 15% | 15% |
| **Système stabilisant :** | | | | | | |
| Polyélectrolyte ($PA_1$) | 1% | 1% | 1% | 1% | 1% | 1,25% |
| Gomme de Tara(2) | 1% | 1% | 1% | 1% | 1% | 0,75% |
| **Phase aqueuse :** | | | | | | |
| Eau | Qs.100% | Qs.100% | Qs.100% | Qs.100% | Qs.100% | Qs.100% |
| Chlorure de sodium | 2% | 4% | 6% | 8% | 10% | 2% |
| Geogard™ 221(1) | 0,6% | 0,6% | 0,6% | 0,6% | 0,6% | 0,6% |

**Tableau 1**

| Emulsion | ($E_7$) | ($E_8$) | ($E_9$) | ($E_{10}$) | ($E_{11}$) |
|---|---|---|---|---|---|
| **Phase grasse :**<br>Triglycérides C8-C10 | 15% | 15% | 15% | 15% | 15% |
| **Système stabilisant :**<br>Polyélectrolyte ($PA_1$)<br>Gomme de Tara (2) | <br>1,25%<br>0,75% | <br>1,25%<br>0,75% | <br>0,5%<br>1,50% | <br>0,5%<br>1,50% | <br>1,50%<br>0,5% |
| **Phase aqueuse :**<br>Eau<br>Chlorure de sodium<br>Geogard™ 221(1) | <br>Qs.100%<br>4%<br>0,6% | <br>Qs.100%<br>10%<br>0,6% | <br>Qs. 00%<br>4%<br>0,6% | <br>Qs.100%<br>10%<br>0,6% | <br>Qs.100%<br>4%<br>0,6% |

**Tableau 1 (suite)**

| Emulsion | ($E_{12}$) | ($E_{13}$) |
|---|---|---|
| **Phase grasse :**<br>Triglycérides C8-C10 | 15% | 15% |
| **Système stabilisant :**<br>Polyélectrolyte ($PA_2$)<br>Gomme de Tara (2) | <br>1%<br>1% | <br>1%<br>1% |
| **Phase aqueuse :**<br>Eau<br>Chlorure de sodium<br>Geogard™ 221(1) | <br>Qs. 00%<br>2%<br>0,6% | <br>Qs.100%<br>10%<br>0,6% |

**Tableau 1 (fin)**

(1) : Geogard™ 221 est un mélange d'acide déhydroacétique et d'alcool benzylique util comme agent conservateur et commercialisé par la société LONZA.

(2) : Gomme de Tara (numéro CAS : 39300-88-4) est commercialisée sous l'appellat « Gomme de Tara » par la société Starlight.

**2-2 : Préparation d'émulsions huile-dans-eau selon l'état de la technique.**

On prépare dix émulsions huile-dans-eau, notées ($F_1$) à ($F_{10}$), dont les proportions massiques en leurs constituants sont consignées dans le tableau 2 ci-dessous, en mettant en oeuvre le procédé suivant :

- Dans un premier bécher, on disperse progressivement et successivement à une température de 20°C, l'un des polyélectrolytes $PA_1$ à $PA_4$ et la gomme de Tara dans une phase grasse sous agitation mécanique à 80 tours par minute ;
- On prépare dans un second bécher, à une température de 20°C, la phase aqueuse comprenant l'eau et la quantité massique de sel souhaitée ;
- Le contenu du premier bécher est progressivement versé dans le second bécher à une température de 20°C, sous agitation mécanique au moyen d'une défloculeuse à 1.200 tours par minute ;
- Le mélange obtenu est maintenu sous agitation pendant 10 minutes, puis vidangé pour obtenir les émulsions huile-dans-eau ($F_1$) à ($F_{10}$).

| Emulsion | ($F_1$) | ($F_2$) | ($F_3$) | ($F_4$) | ($F_5$) |
|---|---|---|---|---|---|
| **Phase grasse :**<br>Triglycérides C8-C10 | 15% | 15% | 15% | 15% | 15% |
| **Système stabilisant :**<br>Polyélectrolyte ($PA_1$)<br>Gomme de Tara | 1%<br>1% | 2%<br>0% | 2%<br>0% | 0%<br>2% | 1,25%<br>0,75% |
| **Phase aqueuse :**<br>Eau<br>Chlorure de sodium<br>Geogard™ 221 | Qs.100%<br>0%<br>0,6% | Qs.100%<br>2%<br>0,6% | Qs.100%<br>4%<br>0,6% | Qs.100%<br>4%<br>0,6% | Qs.100%<br>0%<br>0,6% |

**Tableau 2**

| Emulsion | ($F_6$) | ($F_7$) | ($F_8$) | ($F_9$) | ($F_{10}$) |
|---|---|---|---|---|---|
| **Phase grasse :**<br>Triglycérides C8-C10 | 15% | 15% | 15% | 15% | 15% |
| **Système stabilisant :**<br>Polyélectrolyte ($PA_1$)<br>Polyélectrolyte ($PA_3$)<br>Polyélectrolyte ($PA_4$)<br>Gomme de Tara | 0,5%<br>0%<br>0%<br>1,50% | 0%<br>1%<br>0%<br>1% | 0%<br>1%<br>0%<br>1% | 0%<br>0%<br>1%<br>1% | 0%<br>0%<br>1%<br>1% |
| **Phase aqueuse :**<br>Eau<br>Chlorure de sodium<br>Geogard™ 221[(1)] | Qs. 100%<br>0%<br>0,6% | Qs.100%<br>2%<br>0,6% | Qs.100%<br>10%<br>0,6% | Qs. 100%<br>2%<br>0,6% | Qs.100%<br>10%<br>0,6% |

**Tableau 2 (fin)**

**2-3 : <u>Mise en évidence des propriétés et des caractéristiques des émulsions huile-dans-eau selon l'invention comparativement aux émulsions huile-dans-eau de l'état de la technique.</u>**

Les émulsions huile-dans-eau ($E_1$) à ($E_{13}$) selon l'invention et les émulsions huile-dans-eau ($F_1$) à ($F_{10}$) selon l'état de la technique ainsi préparées, sont ensuite conservées dans une enceinte climatique isolée et régulée à une température de 20°C pendant 7 jours. A l'issue de cette période de 7 jours et pour chaque émulsions huile-dans-eau :

- On observe l'aspect visuel,
- On mesure la viscosité dynamique ($\mu$) à 20°C, au moyen d'un viscosimètre de type Brookfield LVT à une vitesse de 6 tours par minute (V6) lorsque ladite viscosité dynamique est inférieure ou égale à 100.000 mPa.s environ, muni du mobile adapté ou au moyen d'un viscosimètre de type Brookfield RVT à une vitesse de 5 tours par minute lorsque ladite viscosité dynamique est supérieure à 100.000 mPa.s, muni d'un mobile adapté, de chaque émulsion.
- On mesure la conductivité à 20°C, au moyen d'un conductimètre de marque LF 196 de la société WTW muni d'une électrode Tétracon™ 96.

Les émulsions huile-dans-eau sont ensuite replacées et conservées dans la même enceinte climatique isolée et régulée à une température de 20°C jusqu'à trois mois. Après une période de trois mois, chaque émulsion est sortie de l'enceinte climatique pour en observer l'aspect. Les résultats obtenus pour les émulsions huile-dans-eau ($E_1$) à ($E_{13}$) selon l'invention sont consignés dans le tableau 3 ci-après et les résultats obtenus pour les émulsions huile-dans-eau comparatives ($F_1$) à ($F_{10}$) sont consignés dans le tableau 4 suivant.

| Emulsion | ($E_1$) | ($E_2$) | ($E_3$) | ($E_4$) | ($E_5$) | ($E_6$) | ($E_7$) |
|---|---|---|---|---|---|---|---|
| Aspect visuel après 7 jours à 20°C | ++ | ++ | ++ | ++ | ++ | ++ | ++ |
| Viscosité (Brookfield LVT, V6) en mPas. | 83.000 | 75.000 | 78.000 | 71.000 | 82.000 | 86.000 | 62.500 |
| Conductivité en $mS.cm^{-1}$ | 32,6 | 54,8 | 76,6 | 96,5 | 116,5 | 31,8 | 55,0 |
| Aspect visuel après 3 mois à 20°C | ++ | ++ | ++ | ++ | ++ | ++ | ++ |

**Tableau 3**

| Emulsion | ($E_8$) | ($E_9$) | ($E_{10}$) | ($E_{11}$) | ($E_{12}$) | ($E_{13}$) |
|---|---|---|---|---|---|---|
| Aspect visuel après 7 jours à 20°C | ++ | ++ | ++ | ++ | ++ | ++ |
| Viscosité (Brookfield LVT, V6) en mPas. | 68.000 | 95.000 | 124.000* | 54.000 | 84.000 | 90.000 |
| Conductivité en $mS.cm^{-1}$ | 113,9 | 55,1 | 113,4 | 56,5 | 32,3 | 120,4 |
| Aspect visuel après 3 mois à 20°C | ++ | ++ | ++ | ++ | ++ | ++ |

**Tableau 3 (fin)**

+ + : Aspect homogène et lisse

* : viscosité dynamique mesurée à 20°C avec le viscosimètre Brookfield RVT, vitesse 5 tours par minute.

| Emulsion | ($F_1$) | ($F_2$) | ($F_3$) | ($F_4$) | ($F_5$) |
|---|---|---|---|---|---|
| Aspect visuel après 7 jours à 20°C | (-) | (-) | (-) | (-) | (-) |
| Viscosité (Brookfield LVT, V6) en mPas. | 175.000* | 72.500 | 55.000 | 47.000 | 133.000* |
| Conductivité en $mS.cm^{-1}$ | 1,4 | 31,6 | 57,0 | 55,4 | 1,7 |
| Aspect visuel après 3 mois à 20°C | (--) | (--) | (--) | (--) | (--) |

**Tableau 4**

| Emulsion | (F6) | (F7) | (F8) | (F9) | (F10) |
|---|---|---|---|---|---|
| Aspect visuel après 7 jours à 20°C | (-) | (-) | (-) | (-) | (-) |
| Viscosité (Brookfield LVT, V6) en mPas. | 142.000* | 33.000 | 41.000 | 31.000 | 40.000 |
| Conductivité en $mS.cm^{-1}$ | 0,7 | 31,4 | 120,7 | 31,5 | 120,6 |
| Aspect visuel après 3 mois à 20°C | (--) | (--) | (--) | (--) | (--) |

**Tableau 4 (fin)**

* : viscosité dynamique mesurée à 20°C avec le viscosimètre Brookfield RVT, vitesse 5 tours par minute.

(-) : Présence de grumeaux et d'amas

(--) : Aspect hétérogène avec présence de grumeaux et d'amas

**2-4 : <u>Analyse des résultats.</u>**

Les résultats sont jugés satisfaisants lorsque l'aspect visuel d'une émulsion huile-dans-eau est jugé homogène et lisse après une durée de stockage de trois mois à 20°C de ladite émulsion huile-dans-eau, et lorsque sa viscosité dynamique mesurée à 20°C, au moyen d'un viscosimètre de type Brookfield LVT à une vitesse de 6 tours par minutes, muni du mobile adapté, est supérieure ou égale à 30.000mPa.s

Les émulsions ($E_1$) à ($E_{13}$) selon l'invention présentent un aspect lisse, dépourvu de grumeaux et d'amas, même à l'issu d'une période de stockage prolongée de 3 mois à 20°C.

Au contraire, les résultats obtenus avec les émulsions ($F_2$) et ($F_3$), font apparaître que, lorsque le système stabilisant de l'émulsion huile-dans-eau est constitué du seul polyélectrolyte ($PA_1$) en présence d'une quantité de 2% et de 4% de chlorure de sodium, on n'obtient pas d'émulsions huile-dans-eau présentant un aspect homogène et lisse après une période de stockage de 7 jours à 20°C.

De plus les résultats obtenus avec l'émulsion ($F_4$) font apparaître que lorsque le système stabilisant de l'émulsion huile-dans-eau est constitué de la seule gomme de Tara en présence de 4% de NaCl, on n'obtient pas d'émulsions huile-dans-eau présentant un aspect homogène et lisse après une période de stockage de 7 jours à 20°C.

Pour des ratios massiques «gomme de tara » sur polyélectrolyte ($PA_1$) respectivement égaux à 1/1, à 3/5 et à 3/1 et en absence de chlorure de sodium, les résultats obtenus avec les émulsions ($F_1$), ($F_5$) et ($F_6$) font apparaître que l'on n'obtient pas d'émulsions huile-dans-eau présentant un aspect homogène et lisse après une période de stockage de 7 jours à 20°C.

Les résultats obtenus avec les émulsions ($F_7$) et ($F_8$), ($F_9$) et ($F_{10}$) comprenant respectivement 2% massique et 4% massique de chlorure de sodium, font apparaître que lorsque le système stabilisant est constitué par la gomme de Tara et l'un des polyélectrolytes ($PA_3$) ou ($PA_4$), on n'obtient pas d'émulsions huile-dans-eau présentant un aspect homogène et lisse après une période de stockage de 7 jours à 20°C.

La comparaison des résultats obtenus avec les émulsions huile-dans-eau ($E_1$) à ($E_{13}$) selon l'invention avec ceux obtenus avec les émulsions huile-dans-eau ($F_1$) à ($F_6$) selon l'état de la technique, permet de mettre en évidence une amélioration de l'aspect des émulsions huile-dans-eau riches en sel, et tout en conservant un niveau élevé de viscosité, constitutive d' un effet technique supplémentaire induit par l'invention objet de la présence demande de brevet.

**3-1 : <u>Préparation d'émulsions huile-dans-eau selon l'état de la technique</u>**

On prépare deux émulsions huile-dans-eau, notées ($F_{11}$) et ($F_{12}$), dont les proportions massiques en leurs constituants sont consignées dans le tableau 5 ci-dessous, en mettant en oeuvre le procédé suivant :

- Dans un premier bécher, on disperse progressivement et successivement à une température de 20°C, le polyélectrolyte $PA_1$ et la gomme de Xanthane dans une phase grasse sous agitation mécanique à 80 tours par minute ;
- On prépare dans un second bécher, à une température de 20°C, la phase aqueuse comprenant l'eau et la quantité massique de sel souhaitée ;
- Le contenu du premier bécher est progressivement versé dans le second bécher à une température de 20°C, sous

agitation mécanique au moyen d'une défloculeuse à 1.200 tours par minute ;

- Le mélange alors obtenu est maintenu sous agitation pendant 10 minutes, puis vidangé pour obtenir les émulsions huile-dans-eau ($F_{11}$) et ($F_{12}$).

**Tableau 5**

| **Emulsion** | **($F_{11}$)** | **($F_{12}$)** |
|---|---|---|
| **Phase grasse :**<br>Triglycérides C8-C10 | <br>15% | <br>15% |
| **Système stabilisant :**<br>Polyélectrolyte ($PA_1$)<br>Keltrol™ CG-T(3) | <br>1%<br>1% | <br>1%<br>1% |
| **Phase aqueuse :**<br>Eau<br>Chlorure de sodium<br>Geogard™ 221 | <br>Qs.100%<br>2%<br>0,6% | <br>Qs.100%<br>10%<br>0,6% |
| (3) : Keltrol™ CG-T est de la gomme de xanthane commercialisée par la société CP Kelco | | |

**3-2 : Préparation d'émulsions huile-dans-eau de type masque crème « rince off » restructurant pour cheveux stressés et fragilisés selon l'invention et selon l'état de la technique**

On prépare une émulsion huile-dans-eau selon l'état de la technique notée ($F_{13}$) et une émulsion huile-dans-eau selon l'invention notée ($E_{14}$), dont les proportions massiques en leurs constituants sont consignées dans le tableau 6 ci-dessous, en mettant en oeuvre le procédé suivant :

- Dans un premier bécher, on prépare une phase grasse en introduisant successivement du Lanol™P, du Lanol™99, de l'huile de jojoba et du Montanov™82 à une température de 80°C, puis on disperse progressivement le polyélectrolyte $PA_1$ puis la gomme de Tara ou la gomme de xanthane sous agitation mécanique à 80 tours par minute ;
- On prépare dans un second bécher, à une température de 20°C, la phase aqueuse comprenant de l'eau, sur laquelle on verse de façon progressive et successive le butylène glycol, le N-cocoyl aminoacides, le PECOSIL™SPP 50, l'AMONYL™DM, le SEPICIDE™HB et le SEPICIDE™CI ;
- Le contenu du premier bécher est progressivement versé dans le second bécher à une température de 80°C, sous agitation mécanique au moyen d'une défloculeuse à 1.200 tours par minute ;
- Le mélange obtenu est maintenu sous agitation pendant 10 minutes, puis vidangé pour obtenir les émulsions huile-dans-eau ($F_{13}$) et ($E_{14}$).

**Tableau 6**

| **Emulsion** | **($F_{13}$)** | **($E_{14}$)** |
|---|---|---|
| **Phase grasse :**<br>Huile de jojoba<br>Lanol™ P(4)<br>Lanol™ 99(5)<br>Montanov™ 82(6) | <br>1%<br>6%<br>5%<br>3% | <br>1%<br>6%<br>5%<br>3% |
| **Système stabilisant :**<br>Polyélectrolyte (PA1)<br>Keltrol™ CG-T(3)<br>Gomme de Tara(2) | <br>1%<br>1%<br>0% | <br>1%<br>0%<br>1% |
| **Phase aqueuse :**<br>Eau<br>Butylène Glycol | <br>Qs.100%<br>3% | <br>Qs.100%<br>3% |

(suite)

| Emulsion | ($F_{13}$) | ($E_{14}$) |
|---|---|---|
| N-cocoyl aminoacides | 0,7% | 0,7% |
| PECOSIL™ SPP 50(7) | 0,75% | 0,75% |
| AMONYL™ DM(8) | 1% | 1% |
| SEPICIDE™ HB(9) | 0,3% | 0,3% |
| SEPICIDE™ CI(10) | 0,2% | 0,2% |

(4) : le LANOL™ P est un palmitate de glycol utilisé comme additif à effet stabilisant, et commercialisé par la société SEPPIC.
(5) : le LANOL™ 99 est de l'isononanoate d'isononyle commercialisé par la société SEPPIC.
(6) : le MONTANOV™ 82 est un agent émulsionnant à base d'alcool cétéarylique et de cocoylglucoside.
(7) : le PECOSIL™ SPP 50 est un phosphate de potassium de panthényl diméthicone PEG-7, commercialisé par la société PHOENIX.
(8) : l'AMONYL™ DM est un tensioactif cationique se présentant sous la forme d'un sel quaternaire d'ammonium, dont le nom INCI est «Polyquaternium 82 », et commercialisé par la société SEPPIC.
(9) : le SEPICIDE™ HB, qui est un mélange de phénoxyéthanol, de méthylparaben, d'éthylparaben, de propylparaben et de butylparaben, est un agent conservateur commercialisé par la société SEPPIC.
(10) : le SEPICIDE™ CI, imidazolidine urée, est un agent conservateur commercialisé par la société SEPPIC.

**3-3 : Mise en évidence des propriétés et des caractéristiques des émulsions huile-dans-eau selon l'invention comparativement aux émulsions huile-dans-eau de l'état de la technique.**

Les émulsions huile-dans-eau ($F_{11}$), ($F_{12}$) et ($F_{13}$) selon l'état de la technique et l'émulsion ($E_{14}$) selon l'invention sont évaluées selon le protocole expérimental décrit au paragraphe 2-3 précédent.

Les résultats obtenus avec les émulsions huile-dans-eau ($F_{11}$), ($F_{12}$) et ($F_{13}$) selon l'état de la technique et les résultats obtenus avec l'émulsion ($E_{14}$) selon l'invention sont consignés dans le tableau 7 ci-dessous.

**Tableau 7**

| Emulsion | ($F_{11}$) | ($F_{12}$) | ($F_{13}$) | ($E_{14}$) |
|---|---|---|---|---|
| Aspect visuel après 7 jours à 20°C | (-) | (--) | (-) | (++) |
| Viscosité (Brookfield LVT, V6) en mPas. | 56.500 | 52.000 | 300.000* | 305.000* |
| Conductivité en $mS.cm^{-1}$ | 32,8 | 120,6 | 3,7 | 3,5 |
| Aspect visuel après 3 mois à 20°C | (--) | (--) | (--) | (++) |

(+ +) : Aspect homogène et lisse
(-) : Présence de grumeaux et d'amas
(--) : Aspect hétérogène avec présence de grumeaux et d'amas
* : viscosité dynamique mesurée à 20°C avec le viscosimètre Brookfield RVT, vitesse 5 tours par minute.

**3-4 : Analyse des résultats.**

Les résultats sont jugés satisfaisants lorsque l'aspect visuel d'une émulsion huile-dans-eau est jugé homogène et lisse après une durée de stockage de trois mois à 20°C de ladite émulsion huile-dans-eau, et lorsque sa viscosité dynamique mesurée à 20°C, au moyen d'un viscosimètre de type Brookfield LVT à une vitesse de 6 tours par minutes, muni du mobile adapté, est supérieure ou égale à 30.000mPa.s.

Les émulsions ($F_{11}$) et ($F_{12}$) selon l'état de la technique doivent être respectivement comparées aux émulsions ($E_1$) et ($E_5$) selon l'invention car elles ne diffèrent dans leurs constitutions que par la mise en oeuvre de la gomme de xanthane à la place de la gomme de Tara dans le système stabilisant. Les émulsions ($E_1$) et ($E_5$) présentent un aspect lisse, dépourvu de grumeaux et d'amas, à l'issue d'une période de stockage prolongée de 3 mois à 20°C, alors que les émulsions ($F_{11}$) et ($F_{12}$) présentent un aspect hétérogène avec présence de grumeaux et d'amas après la même période de stockage dans les mêmes conditions opératoires.

De même, l'émulsion huile-dans-eau ($E_{14}$) selon l'invention présente un aspect lisse, dépourvu de grumeaux et d'amas, à l'issue d'une période de stockage prolongée de 3 mois à 20°C, alors que l'émulsion huile-dans-eau ($F_{13}$)

selon l'état de la technique présente un aspect hétérogène avec présence de grumeaux et d'amas après la même période de stockage dans les mêmes conditions opératoires.

La comparaison des résultats obtenus avec les émulsions huile-dans-eau ($E_1$), ($E_5$) et ($E_{14}$) selon l'invention avec ceux obtenus avec les émulsions huile-dans-eau ($F_{11}$), ($F_{12}$) et ($F_{13}$) selon l'état de la technique, permet aussi de mettre en évidence une amélioration de l'aspect des émulsions huile-dans-eau riches en sel, et tout en conservant un niveau élevé de viscosité, constitutive d' un effet technique supplémentaire induit par l'invention objet de la présence demande de brevet.

<u>**Exemple de formules illustratives**</u>

**4-1 : Gel-crème soin hydratant corporel**

<u>FORMULE :</u>

| | | |
|---|---|---|
| A | Huile de Jojoba | 14,10% |
| | C12-C15 alkyl Benzoate | 6,7% |
| | DC 245 | 4,2% |
| | DL alpha Tocophérol | 0,05% |
| B | Maris Aqua | 70,85% |
| | AQUAXYL™ | 3% |
| C | Polyélectrolyte ($PA_1$) | 2% |
| | Gomme de Tara$^{(2)}$ | 1% |
| D | Euxyl™ PE9010 | 1% |
| | Fragrance | 0,1% |

<u>MODE OPERATOIRE:</u>

Mélanger les constituants de la phase grasse A à une température de 80°C sous agitation. Y ajouter ensuite successivement les ingrédients de la phase C.

Préparer la phase aqueuse B et la chauffer à 80°C sous agitation.

Ajouter la phase aqueuse B de façon progressive sur le mélange des phases A+C puis émulsionner à l'aide d'un agitateur muni d'un mobile rotor-stator de marque Silverson.

Refroidir ensuite à 25°C, puis ajouter la phase D.

Aspect après 1 jour à 20°C : Crème compacte homogène.
Viscosité dynamique après 1 jour à 20°C : 117.000 mPa.s (Brookfield RVT, M7, V5) Aspect après 7 jours à 20°C: Crème compacte homogène.
Viscosité dynamique après 7 jours à 20°C : 105.000 mPa.s (Brookfield RVT, M7, V5) Aspect après 1 mois à 20°C: Crème compacte homogène.
Viscosité dynamique après 1 mois à 20°C : 110.000 mPa.s (Brookfield RVT, M7, V5).

**4-2: Gel-crème Masque visage.**

<u>FORMULE :</u>

| | | |
|---|---|---|
| A | Triglycérides 4555 (C8C10) | 9% |
| | C12-C15 alkyl Benzoate | 4% |
| | Isohexadécane | 2% |
| | DL alpha Tocopherol | 0,10% |
| B | Maris Aqua | qsp 100% |
| C | Polyélectrolyte ($PA_1$) | 1,3% |

(suite)

| | | |
|---|---|---|
| | Gomme de Tara(2) | 0,7% |
| D | Euxyl PE9010 | 1% |
| | Fragrance | 0,1% |

MODE OPERATOIRE :

Mélanger les constituants de la phase grasse A à une température de 80°C sous agitation. Y Ajouter ensuite successivement les ingrédients de la phase C.
Préparer la phase aqueuse B et la chauffer à 80°C sous agitation.
Ajouter la phase aqueuse B de façon progressive sur le mélange des phases A+C puis émulsionner à l'aide d'un agitateur muni d'un mobile rotor-stator de marque Silverson.
Refroidir ensuite à 25°C et ajouter la phase D.

Aspect après 1 jour à 20°C: Crème compacte homogène.
Viscosité dynamique après 1 jour à 20°C : 71.000 mPa.s (Brookfield LVT, M4 V6) Aspect après 7 jours à 20°C: Crème compacte homogène.
Viscosité dynamique après 7 jours à 20°C : 78.400 mPa.s (Brookfield LVT, M4 V6) Aspect après 1 mois à 20°C: Crème compacte homogène.
Viscosité dynamique après 1 mois à 20°C : 79.100 mPa.s (Brookfield LVT, M4 V6)

**4-3 : Crème corporelle.**

FORMULE :

| | |
|---|---|
| Triglycérides 4555 (C8C10) | 12% |
| C12-C15 alkyl Benzoate | 5,3% |
| Isohexadécane | 2,7% |
| Alcool cétylique | 2% |
| DL alpha Tocophérol | 0,10% |
| Polyélectrolyte ($PA_1$) | 1,5% |
| Gomme de Tara(2) | 0,5% |
| Eau | qsp 100% |
| Givobio™ GZn | 1% |
| Sepicalm™ S | 3% |
| Euxyl™ PE9010 | 1% |
| Fragrance | 0,1% |

**4-4 : Spray solaire organo-minéral**

FORMULE:

| | | |
|---|---|---|
| A | Isodecyl neopentanoate | 20% |
| | Cyclodiméthicone | 5% |
| | Ethylhexylmethoxicinnamate | 6% |
| | Butyl Methoxydibenzoylmethane | 3% |
| | DL alpha Tocopherol | 0.05% |
| B | Eau | qsp 100% |
| | EDTA tétrasodique | 0,2% |
| | Glycérine | 7% |

(suite)

| | | |
|---|---|---|
| | Phenyl Benzyimidazole Sulfonic Acid (salifié avec quantité molaire nécessaire de soude) | 3% |
| C | Polyélectrolyte ($PA_1$) | 1,3% |
| | Gomme de Tara(2) | 0,7% |
| D | SEPICIDE™ HB | 1% |
| | Fragrance | 0,1% |

AQUAXYL™ (nom INCI : Xylitylglucoside & Anhydroxylitol & Xylitol) : Composition hydratante commercialisée par la société SEPPIC.
Euxyl™ PE9010 (Nom INCI : Phenoxyethanol & Ethylhexyl Glycerin) : Composition utilisée comme agent conservateur.
GIVOBIO™ GZn (nom INCI : Zinc Gluconate) : Composition commercialisée par la société SEPPIC.
LANOL™ 99 (nom INCI : Isononyle Isononanoate) : Ester utilisé comme phase huileuse dans la préparation de composition cosmétique et distribué par la société SEPPIC.
Maris Aqua : eau de mer à 8% de chlorure de sodium.
SEPICIDE™ HB (nom INCI: Phenoxyethanol / Methylparaben / Ethylparaben /
Propylparaben / Butylparaben) : Agent conservateur contenant du phénoxyéthanol, commercialisé par la société SEPPIC.
SEPICALM™ S : (nom INCI : Sodium Cocoyl Aminoacids And Sarcosine And Potassium
Aspartate And Magnésium Aspartate) : Composition anti-inflammatoire commercialisée par la société SEPPIC.
SERENIKS™ 207 (nom INCI : Tsuga Canadensis Leaf Extract And Water And Butylene Glycol) est une composition anti-âge.

## Revendications

**1.** Composition ($C_1$) se présentant sous la forme d'une émulsion de type huile-dans-eau, caractérisée en ce qu'elle comprend pour 100% de sa masse :

- De 5% à 55% massique d'une phase grasse ($P_1$) constituée d'au moins une huile et optionnellement d'au moins une cire,
- De 0,025% à 3,75% massique d'au moins un polyélectrolyte anionique réticulé (PA) issu de la polymérisation de l'acide 2-méthyl 2-[(1-oxo 2-propènyl) amino] 1-propanesulfonique partiellement ou totalement salifiée, avec au moins un monomère neutre choisi parmi l'acrylate de (2-hydroxy éthyle) et le N,N-diméthyl acrylamide et au moins un monomère de formule (I) :

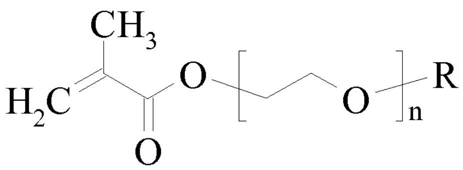

(I)

dans laquelle R représente un radical alkyle linéaire ou ramifié comportant de huit à vingt atomes de carbone et n représente un nombre supérieur ou égal à un et inférieur ou égal à vingt, en présence d'au moins un agent de réticulation,
- De 0,025% à 3,75% massique d'au moins un galactomannane (GM) provenant de gomme de tara et présentant un degré de substitution (DS) d'environ 1/3,
- De 37,5% à 94,95% massique d'une phase aqueuse ($P_2$) cosmétiquement acceptable, ladite phase aqueuse ($P_2$) comprenant pour 100% de sa masse de 1% à 15% massique d'au moins un sel (S) se présentant sous une forme dissoute,

ladite composition ($C_1$) étant en outre **caractérisée en ce que** le rapport massique entre le galactomannane (GM) et le polyélectrolyte anionique réticulé (PA) est supérieur ou égal à 1/3 et inférieur ou égal à 3/1 ; et **en ce que** ledit polyélectrolyte anionique réticulé (PA) comporte pour 100% molaire de ses monomères constitutifs :
- De 20% molaire à 80% molaire d'unités monomériques issues dudit acide 2-méthyl 2-[(1-oxo 2-propènyl)

amino] 1-propanesulfonique partiellement ou totalement salifiée ;
- De 15% molaire à 75% molaire d'unités monomériques issues dudit monomère neutre;
- De 0,5% à 5% molaire d'unités monomériques issues dudit monomère de formule (I).

**2.** Composition ($C_1$) telle que définie à la revendication 1, **caractérisée en ce que** ledit monomère de formule (I) est le méthacrylate de lauryle tétraéthoxylé.

**3.** Composition ($C_1$) telle que définie à l'une ou quelconque des revendications 1 ou 2, **caractérisée en ce que** ledit polyélectrolyte anionique réticulé (PA) est un terpolymère de l'acide 2-méthyl 2- [(1-oxo 2-propènyl) amino] 1-propanesulfonique partiellement salifié sous forme de sel d'ammonium, du N,N-diméthyl acrylamide et du méthacrylate de lauryle tétraéthoxylé, réticulé au triméthylol propanetriacrylate.

**4.** Composition ($C_1$) telle que définie à l'une ou quelconque des revendications 1 ou 2, **caractérisée en ce que** ledit polyélectrolyte anionique réticulé (PA) est un terpolymère de l'acide 2-méthyl 2- [(1-oxo 2-propènyl) amino] 1-propanesulfonique partiellement salifié sous forme de sel d'ammonium, de l'acrylate de (2-hydroxy éthyle) et du méthacrylate de lauryle tétraéthoxylé, réticulé au triméthylol propanetriacrylate.

**5.** Composition ($C_1$) telle que définie à l'une ou quelconque des revendications 1 à 4 **caractérisée en ce que** le sel (S) est un sel inorganique constitué par un cation qui est l'ion ammonium ou un cation métallique et par un anion sélectionné parmi les éléments du groupe constitué par les halogénures, les carbonates, les bicarbonates, les phosphates, les nitrates, les borates et les sulfates.

**6.** Composition ($C_1$) telle que définie à l'une des revendications 1 à 4, **caractérisée en ce que** le sel (S) est un sel organique constitué par un cation qui est l'ion ammonium ou un cation métallique et par un anion organique qui est un composé organique possédant au moins une fonction acide carboxylique sous forme carboxylate ou au moins une fonction acide sulfonique sous forme sulfonate ou au moins une fonction sulphate.

**7.** Composition ($C_1$) telle que définie à la revendication 6, **caractérisée en ce que** le sel (S) est un sel organique sélectionné parmi les éléments du groupe constitué par le glycolate de sodium, le citrate de sodium, le salicylate de sodium, le lactate de sodium, le gluconate de sodium, le gluconate de zinc, le gluconate de manganèse, le gluconate de cuivre et l'aspartate de magnésium.

**8.** Composition ($C_1$) telle que définie à la revendication 6 **caractérisée en ce que** le sel (S) est un sel organique sélectionné parmi les éléments du groupe constitué par le 2-phénylbenzimidazole-5-sulphonate de sodium et le 4-hydroxy 2-méthoxy 5-(oxo-phénylméthyl)benzènesulphonate de sodium.

**9.** Composition ($C_1$) telle que définie à l'une ou quelconque des revendications 1 à 8, **caractérisée en ce que** sa viscosité dynamique mesurée à une température de 20°C, au moyen d'un viscosimètre de type Brookfield est supérieure ou égale à 30.000mPa.s et inférieure ou égale à 200.000mPa.s

**10.** Procédé de préparation d'une composition ($C_1$) telle que définie à l'une ou quelconque des revendications 1 à 9, **caractérisé en ce qu'**il comprend :

Au moins une étape a) de préparation d'une phase ($P'_1$) par mélange du polyélectrolyte anionique réticulé (PA) et du galactomannane (GM) dans la phase grasse ($P_1$); et
Au moins une étape b) d'émulsification de ladite phase ($P'_1$) obtenue à l'issue de l'étape a) avec la phase aqueuse ($P_2$) cosmétiquement acceptable.

**11.** Utilisation cosmétique de la composition ($C_1$) telle que définie à l'une ou quelconque des revendications 1 à 9, pour le nettoyage, pour la protection et/ou pour le soin de la peau, des cheveux, du cuir chevelu ou des muqueuses.

## Patentansprüche

**1.** Zusammensetzung ($C_1$), die die Form einer Emulsion vom Typ Öl-in-Wasser aufweist, **dadurch gekennzeichnet, dass** sie zu 100 % ihrer Masse folgendes umfasst:

- von 5 Massen-% bis 55 Massen-% eine fette Phase ($P_1$), bestehend aus mindestens einem Öl und wahlweise

aus mindestens einem Wachs,
- von 0,025 Massen-% bis 3,75 Massen-% mindestens einen vernetzten anionischen Polyelektrolyten (PA), stammend aus der Polymerisation von teilweise oder ganz in der Form von Salzen vorliegender 2-Methyl-2-[(1-oxo-2-propenyl)amino]-1-propansulfonsäure mit mindestens einem neutralen Monomer, gewählt aus 2-Hydroxyethylacrylat und N,N-Dimethylacrylamid, und mindestens einem Monomer der Formel (I):

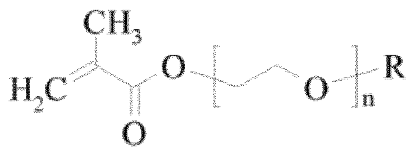

(I)

wobei R einen linearen oder verzweigten, von acht bis zwanzig Kohlenstoffatome enthaltenden, Alkylrest darstellt und n eine Zahl größer als oder gleich eins und kleiner als oder gleich zwanzig darstellt, in Gegenwart von mindestens einem Verzweigungsmittel,
- von 0,025 Massen-% bis 3,75 Massen-% mindestens ein aus Tarakernmehl stammendes und einen Substitutionsgrad (DS) von ungefähr 1/3 aufweisendes Galaktomannan (GM),
- von 37,5 Massen-% bis 94,95 Massen-% eine kosmetisch annehmbare wässrige Phase ($P_2$), wobei die wässrige Phase ($P_2$) zu 100 % seiner Masse von 1 Massen-% bis 15 Massen-% von mindestens einem, eine gelöste Form aufweisenden, Salz (S) umfasst, wobei die Zusammensetzung ($C_1$) des Weiteren **dadurch gekennzeichnet ist, dass** das Massenverhältnis zwischen dem Galaktomannan (GM) und dem verzweigten anionischen Polyelektrolyten (PA) größer oder gleich 1/3 und kleiner oder gleich 3/1 ist; und dadurch, dass der vernetzte anionische Polyelektrolyt (PA) zu 100 Mol-% seine konstitutiven Monomere enthält:
- von 20 Mol-% bis 80 Mol-% aus der 2-Methyl-2-[(1-oxo-2-propenyl)-amino]-1-propansulfonsäure, teilweise oder ganz in der Form von Salzen vorliegend, stammende Monomereinheiten;
- von 15 Mol-% bis 75 Mol-% aus dem neutralen Monomer stammende Monomereinheiten;
- von 0,5 Mol-% bis 5 Mol-% aus dem Monomer der Formel (I) stammende Monomereinheiten.

2. Zusammensetzung ($C_1$) nach Anspruch 1, **dadurch gekennzeichnet, dass** das Monomer der Formel (I) tetraethoxyliertes Laurylmethacrylat ist.

3. Zusammensetzung ($C_1$) nach einem oder beliebigen der Ansprüche 1 oder 2, **dadurch gekennzeichnet, dass** der vernetzte anionische Polyelektrolyt (PA) ein Terpolymer der 2-Methyl-2-[(1-oxo-2-propenyl)-amino]-1-propansulfonsäure, teilweise oder ganz in der Form von Salzen vorliegend, in der Form des Ammoniumsalzes, des N,N-Dimethylacrylamids und des tetraethoxylierten Laurylmethacrylats ist, vernetzt mit Trimethylolpropantriacrylat.

4. Zusammensetzung ($C_1$) nach einem oder beliebigen der Ansprüche 1 oder 2, **dadurch gekennzeichnet, dass** der vernetzte anionische Polyelektrolyt (PA) ein Terpolymer der 2-Methyl-2-[(1-oxo-2-propenyl)-amino]-1-propansulfonsäure, teilweise oder ganz in der Form von Salzen vorliegend, in der Form des Ammoniumsalzes, des 2-Hydroxyethylacrylats und des tetraethoxylierten Laurylmethacrylats ist, vernetzt mit Trimethylolpropantriacrylat.

5. Zusammensetzung ($C_1$) nach einem oder beliebigen der Ansprüche 1 bis 4, **dadurch gekennzeichnet, dass** das Salz (S) ein anorganisches Salz ist, bestehend aus einem Kation, das das Ammoniumion oder ein Metallkation ist, und aus einem Anion, ausgewählt aus den Elementen der Gruppe, bestehend aus Halogenen, Carbonaten, Bicarbonaten, Phosphaten, Nitraten, Boraten und Sulfaten.

6. Zusammensetzung ($C_1$) nach einem der Ansprüche 1 bis 4, **dadurch gekennzeichnet, dass** das Salz (S) ein organisches Salz ist, bestehend aus einem Kation, das das Ammoniumion oder ein Metallkation ist, und aus einem organischen Anion, das eine organische Verbindung ist, die mindestens eine Carbonsäurefunktion in Carboxylatform oder mindestens eine Sulfonsäurefunktion in Sulfonatform oder mindestens eine Sulfatfunktion besitzt.

7. Zusammensetzung ($C_1$) nach Anspruch 6, **dadurch gekennzeichnet, dass** das Salz (S) ein organisches Salz ist, ausgewählt aus den Elementen der Gruppe, bestehend aus Natriumglycolat, Natriumcitrat, Natriumsalicylat, Natriumlactat, Natriumgluconat, Zinkgluconat, Mangangluconat, Kupfergluconat und Magnesiumaspartat.

8. Zusammensetzung ($C_1$) nach Anspruch 6, **dadurch gekennzeichnet, dass** das Salz (S) ein organisches Salz ist, ausgewählt aus den Elementen der Gruppe, bestehend aus Natrium-2-phenylbenzimidazol-5-sulfonat und Natrium-4-hydroxy-2-methoxy-5-(oxo-phenylmethyl)benzolsulfonat.

**9.** Zusammensetzung ($C_1$) nach einem oder beliebigen der Ansprüche 1 bis 8, **dadurch gekennzeichnet, dass** ihre bei einer Temperatur von 20 °C mittels eines Viskosimeters vom Typ Brookfield gemessene dynamische Viskosität größer oder gleich 30.000 mPa.s und kleiner oder gleich 200.000 mPa.s ist.

**10.** Zubereitungsverfahren einer Zusammensetzung ($C_1$) nach einem oder beliebigen der Ansprüche 1 bis 9, **dadurch gekennzeichnet, dass** sie Folgendes umfasst:

mindestens einen Schritt a) der Zubereitung einer Phase ($P'_1$) durch Mischen von vernetztem anionischen Polyelektrolyt (PA) und von Galaktomannan (GM) in die fette Phase ($P_1$); und
mindestens einen Schritt b) des Emulgierens der am Ende des Schritts a) erhaltenen Phase ($P'_1$) mit der kosmetisch annehmbaren wässrigen Phase ($P_2$).

**11.** Kosmetische Verwendung der Zusammensetzung ($C_1$) nach einem oder beliebigen der Ansprüche 1 bis 9, zur Reinigung, zum Schutz und/oder zur Pflege der Haut, der Haare, der Kopfhaut oder der Schleimhäute.

## Claims

**1.** Composition ($C_1$) having the form of an emulsion of the oil-in-water type, **characterised in that** it comprises for 100% of the weight thereof:

- From 5 to 55 wt % of an oil phase ($P_1$) constituted of at least one oil and optionally of at least one wax,
- From 0.025 to 3.75 wt % of at least one cross-linked anionic polyelectrolyte (PA) resulting from the polymerization of partially or completely salified 2-methyl 2-[(1-oxo 2-propenyl) amino] 1-propanesulfonic acid, with at least one neutral monomer selected from (2-hydroxy ethyl) acrylate and N,N-dimethyl acrylamide and at least one monomer of formula (I):

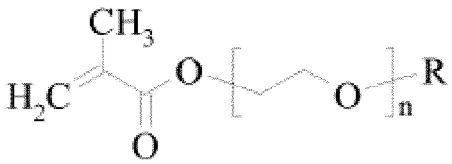

(I)

wherein R is a linear or branched alkyl radical comprising eight to twenty carbon atoms and n is a number greater than or equal to one and less than or equal to twenty, in the presence of at least one cross-linking agent,
- From 0.025 to 3.75 wt % of at least one galactomannan (GM) coming from tara gum and having a degree of substitution (DS) of approximately 1/3,
- From 37.5 to 94.95 wt % of a cosmetically acceptable aqueous phase ($P_2$), said aqueous phase ($P_2$) including, for 100% of the weight thereof from 1% to 15 wt % of at least one salt (S) having a dissolved form,
said composition ($C_1$) being furthermore **characterised in that** the weight ratio between the galactomannan (GM) and the cross-linked anionic polyelectrolyte (PA) is greater than or equal to 1/3 and less than or equal to 3/1; and **in that** said cross-linked anionic polyelectrolyte (PA) comprises for 100 mol% of the constituent monomers thereof:
- From 20 mol% to 80 mol% of monomeric units coming from said partially or totally salified 2-methyl 2-[(1-oxo 2-propenyl) amino] 1-propanesulfonic acid;
- From 15 mol% to 75 mol% of monomeric units coming from said neutral monomer;
- From 0.5% to 5 mol% of monomeric units coming from said monomer of formula (I).

**2.** Composition ($C_1$) such as defined in claim 1, **characterised in that** said monomer of formula (I) is tetraethoxylated lauryl methacrylate.

**3.** Composition ($C_1$) such as defined in any one of claims 1 or 2, **characterised in that** said cross-linked anionic polyelectrolyte (PA) is a terpolymer of the partially salified 2-methyl 2-[(1-oxo 2-propenyl) amino] 1-propanesulfonic acid in the form of an ammonia salt, of N,N-dimethyl acrylamide and of tetraethoxylated lauryl methacrylate, cross-linked with trimethylol propane triacrylate.

4. Composition ($C_1$) such as defined in any one of claims 1 or 2, **characterised in that** said cross-linked anionic polyelectrolyte (PA) is a terpolymer of the partially salified 2-methyl 2-[(1-oxo 2-propenyl) amino] 1-propanesulfonic acid in the form of an ammonia salt, of (2-hydroxy ethyl) acrylate and of tetraethoxylated lauryl methacrylate, cross-linked with trimethylol propane triacrylate.

5. Composition ($C_1$) such as defined in any one of claims 1 to 4 **characterised in that** the salt (S) is an inorganic salt comprised of a cation which is the ammonia ion or a metal cation and by an anion selected from the elements of the group comprising halides, carbonates, bicarbonates, phosphates, nitrates, borates and sulphates.

6. Composition ($C_1$) such as defined in one of claims 1 to 4, **characterised in that** the salt (S) is an organic salt comprised of a cation which is the ammonia ion or a metal cation and by an organic anion which is an organic compound that has at least one carboxylic acid function in the form of carboxylate or at least one sulphonic acid function in the form of sulphonate or at least one sulphate function.

7. Composition ($C_1$) such as defined in claim 6, **characterised in that** the salt (S) is an organic salt selected from the elements of the group comprising sodium glycolate, sodium citrate, sodium salicylate, sodium lactate, sodium gluconate, zinc gluconate, manganese gluconate, copper gluconate and magnesium aspartate.

8. Composition ($C_1$) such as defined in claim 6 **characterised in that** the salt (S) is an organic salt selected from the elements of the group comprising sodium 2-phenylbenzimidazole-5-sulphonate and sodium 4-hydroxy 2-methoxy 5(oxo-phenylmethyl)benzenesulphonate.

9. Composition ($C_1$) such as defined in any one of claims 1 to 8, **characterised in that** the dynamic viscosity thereof measured at a temperature of 20°C, by means of a viscometer of the Brookfield type is greater than or equal to 30,000 mPa.s and less than or equal to 200,000 mPa.s

10. Method for preparing a composition ($C_1$) such as defined in any one of claims 1 to 9, **characterised in that** it comprises:

    At least one step a) of preparing a phase ($P'_1$) by mixing of the cross-linked anionic polyelectrolyte (PA) and of the galactomannan (GM) in the oil phase ($P_1$); and
    At least one step b) of emulsification of said phase ($P'_1$) obtained at the end of the step a) with the cosmetically acceptable aqueous phase ($P_2$).

11. Cosmetic use of the composition ($C_1$) such as defined in any one of claims 1 to 9, for the cleaning, for the protection and/or for the care of skin, hair, scalp or mucous membranes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5373044 A
- US 2798053 A
- EP 0301532 A
- EP 816403 A
- EP 1116733 A
- EP 1069142 A
- EP 1496081 A
- WO 2011030044 A
- JP 4540510 B
- FR 2940111
- US 20110274629 A1
- EP 0152095 A1
- FR 2466273


### Littérature non-brevet citée dans la description


- **DANIEL VOET ; JUDITH G. VOET.** Biochemistry. John Wyley & Sons, 1990, 250